# EUROPEAN PATENT APPLICATION

(11) **EP 4 803 629 A1**
(43) Date of publication of application: **09.09.2026**
(21) Application number: 24884866.5
(22) Date of filing: 30.10.2024
(51) Int. Cl.: C12N 15/62, A61K 39/145, A61K 9/51, A61P 31/16

(54) **MULTIVALENT INFLUENZA MRNA VACCINE**

(30) Priority: 31.10.2023 WO PCT/CN2023/128813; 25.10.2024 WO PCT/CN2024/127528
(71) Applicant: Rinuagene Biotechnology Co., Ltd., Suzhou, Jiangsu 215000 (CN); Rinuagene International HK Limited, Kowloon Hong Kong 999077 (HK)
(72) Inventor: CEN, Shan, Suzhou, Jiangsu 215000 (CN); YI, Dongrong, Suzhou, Jiangsu 215000 (CN); ZHANG, Weiguo, Suzhou, Jiangsu 215000 (CN); DONG, Yijie, Suzhou, Jiangsu 215000 (CN)
(74) Representative: Schulz Junghans Patentanwälte PartGmbB
(86) International application number: PCT/CN2024/128701
(87) International publication number: WO 2025/092866

(57) **Abstract**

Provided is an isolated mRNA molecule, including a nucleotide sequence encoding a chimeric immunogenic polypeptide, where the chimeric immunogenic polypeptide includes an immunogenic fragment of hemagglutinin HA of influenza A H5N1, an immunogenic fragment of hemagglutinin HA of influenza A H1N1, and an immunogenic fragment of hemagglutinin HA of influenza B Victoria linked together. Further provided are a composition and a vaccine including the mRNA, a fusion protein encoded by the mRNA, and a method for inducing an immune response against an influenza virus in a subject using the mRNA, the composition, the vaccine, and the fusion protein.

## Description

### TECHNICAL FIELD

The present invention relates to the field of biomedicine, in particular to an mRNA vaccine having a protective effect against multiple different serotypes of influenza virus.

### BACKGROUND

Influenza virus belongs to the family Orthomyxoviridae and is a type of RNA virus. Influenza virus causes seasonal epidemics and sporadic pandemics in humans. It is estimated that influenza virus results in approximately 3 to 5 million cases of severe respiratory disease and approximately 290,000 to 650,000 deaths globally each year. The World Health Organization (WHO) and national disease control authorities recommend annual influenza vaccination, and influenza vaccines are one of the most effective methods for preventing influenza. However, the surface glycoproteins of influenza virus (hemagglutinin HA and neuraminidase NA), which are common antigens in influenza vaccines, are prone to antigenic drift (see Harrison's Infectious Diseases, 1st Chinese Edition, 2019). Variations in the amino acid sequences of glycoproteins or variations in various glycosyltransferases of influenza virus can lead to substantial differences in glycoproteins and their glycosylation modifications, thereby facilitating the emergence of new virus strains. This propensity of influenza virus to alter its antigenic properties often results in the antigenicity of newly emerging strains being mismatched with the immune protection conferred by existing vaccines, leading to generally low protective efficacy (approximately 40% to 60%) of current influenza vaccines, as well as difficulty in preventing seasonal pandemics.

In recent years, influenza virus epidemics have also shown a trend of cross-infection between animals and humans. Animal influenza viruses such as highly pathogenic avian influenza (HPAI) viruses (e.g., H5, H7) have crossed the species barrier to infect humans, causing high mortality rates. However, current mainstream vaccines are quadrivalent influenza vaccines targeting H1N1, H3N2, B/Victoria, and B/Yamagata, and lack effective protection against highly pathogenic H5N1 and H7N9 infections.

Therefore, there is an urgent need to develop a universal vaccine that elicits effective protection against multiple influenza antigens. To achieve broad-spectrum efficacy of influenza vaccines, it is often necessary to incorporate multiple influenza antigens into the vaccine. However, an increase in the number of influenza antigens poses enormous challenges to vaccine quality and vaccine production processes. Therefore, achieving broad-spectrum protective efficacy against influenza virus with as few antigen components as possible has long been one of the development directions of influenza vaccines.

mRNA technology emerged during the COVID-19 pandemic and represents a rapid-response vaccine development platform for addressing epidemic outbreaks. It utilizes linearized plasmid DNA as a template for synthesis via *in vitro* transcription, which avoids the live cell culture process in traditional vaccine production. Moreover, it features a short production cycle, a simple process, and convenient structural modification, making it particularly suitable for responding to explosive epidemics with rapid mutation rates. Furthermore, compared with traditional vaccines such as inactivated vaccines, live attenuated vaccines, and recombinant subunit vaccines, mRNA vaccines can induce strong humoral immunity and cellular immunity, and may also elicit mucosal immunity depending on the route of administration, making them suitable for use as influenza vaccines. Nevertheless, the loading capacity of a single mRNA molecule is limited, and multivalent mRNA molecules encoding multiple influenza antigen peptides and multivalent influenza mRNA vaccines prepared therefrom have not been reported to date.

### SUMMARY

Through in-depth research, the inventors of this application have discovered that integrating nucleotide sequences encoding immunogenic fragments of hemagglutinin HA from influenza A H5N1 and H1N1, and influenza B Victoria separately into a single mRNA molecule can still induce an immune response against multiple influenza virus subtypes of both influenza A and B simultaneously while significantly reducing the loading capacity of the mRNA, thereby achieving broad-spectrum immunogenicity.

Therefore, one objective of the present invention is to provide an mRNA molecule, preferably an isolated mRNA molecule, including a nucleotide sequence encoding a chimeric immunogenic polypeptide, where the chimeric immunogenic polypeptide includes an immunogenic fragment of hemagglutinin HA of influenza A H5N1, an immunogenic fragment of hemagglutinin HA of influenza A H1N1, and an immunogenic fragment of hemagglutinin HA of influenza B Victoria linked together. In the isolated mRNA molecule disclosed herein, the three immunogenic fragments included in the chimeric immunogenic polypeptide encoded thereby may be linked together in various orders. The linkage orders of the immunogenic fragments include, but are not limited to, including in sequence from the N-terminus to the C-terminus: (1) the immunogenic fragment of hemagglutinin HA of H5N1, the immunogenic fragment of hemagglutinin HA of H1N1, and the immunogenic fragment of hemagglutinin HA of influenza B Victoria; (2) the immunogenic fragment of hemagglutinin HA of H1N1, the immunogenic fragment of hemagglutinin HA of H5N1, and the immunogenic fragment of hemagglutinin HA of influenza B Victoria; (3) the immunogenic fragment of hemagglutinin HA of influenza B Victoria, the immunogenic fragment of hemagglutinin HA of H5N1, and the immunogenic fragment of hemagglutinin HA of H1N1; or (4) the immunogenic fragment of hemagglutinin HA of influenza B Victoria, the immunogenic fragment of hemagglutinin HA of H1N1, and the immunogenic fragment of hemagglutinin HA of H5N1. In a preferred embodiment, the chimeric immunogenic polypeptide includes in sequence from the N-terminus to the C-terminus: (1) a head of hemagglutinin HA of H5N1, a stem of hemagglutinin HA of H1N1, and the immunogenic fragment of hemagglutinin HA of influenza B Victoria; (2) the head of hemagglutinin HA of H1N1, the stem of hemagglutinin HA of H5N1, and the immunogenic fragment of hemagglutinin HA of influenza B Victoria; (3) the immunogenic fragment of hemagglutinin HA of influenza B Victoria, the head of hemagglutinin HA of H5N1, and the stem of hemagglutinin HA of H1N1; or (4) the immunogenic fragment of hemagglutinin HA of influenza B Victoria, the head of hemagglutinin HA of H1N1, and the stem of hemagglutinin HA of H5N1. In a preferred embodiment, the chimeric immunogenic polypeptide includes in sequence from the N-terminus to the C-terminus: (1) the head of hemagglutinin HA of H5N1, the stem of hemagglutinin HA of H1N1, and full-length hemagglutinin HA of influenza B Victoria; (2) the head of hemagglutinin HA of H1N1, the stem of hemagglutinin HA of H5N1, and full-length hemagglutinin HA of influenza B Victoria; (3) full-length hemagglutinin HA of influenza B Victoria, the head of hemagglutinin HA of H5N1, and the stem of hemagglutinin HA of H1N1; or (4) full-length hemagglutinin HA of influenza B Victoria, the head of hemagglutinin HA of H1N1, and the stem of hemagglutinin HA of H5N1.

In the isolated mRNA molecule disclosed herein, the three immunogenic fragments included in the chimeric immunogenic polypeptide encoded thereby may be linked together in various means, including but not limited to direct linkage via, for example, a covalent bond, or linkage via a linker. In some embodiments, the immunogenic fragment of hemagglutinin HA of H5N1 and the immunogenic fragment of hemagglutinin HA of H1N1 are directly linked via a covalent bond. In some embodiments, the immunogenic fragment of hemagglutinin HA of influenza B Victoria is linked to the immunogenic fragment of hemagglutinin HA of H5N1 or H1N1 via a linker. In a preferred embodiment, the linker is a peptide linker. In a preferred embodiment, the linker has a structure of -((G)ₙS)ₘ-, where n is selected from 1, 2, 3, 4, 5, or 6, preferably 4; and m is selected from 1, 2, 3, 4, 5, or 6, preferably 3. In a more preferred embodiment, the linker is a peptide linker having an amino acid sequence set forth in SEQ ID NO: 8.

In some embodiments, the immunogenic fragment of hemagglutinin HA of influenza A H5N1 in the mRNA molecule disclosed herein is a head or a stem of HA. In a preferred embodiment, the head of hemagglutinin HA of H5N1 includes an amino acid sequence as set forth in SEQ ID NO: 4 or an amino acid sequence having at least 80%, 90%, 95%, or 99% sequence identity to SEQ ID NO: 4. In some embodiments, the immunogenic fragment of hemagglutinin HA of influenza A H1N1 in the mRNA molecule disclosed herein is a head or a stem of HA. In a preferred embodiment, the stem of hemagglutinin HA of H1N1 includes an amino acid sequence as set forth in SEQ ID NO: 6 or an amino acid sequence having at least 80%, 90%, 95%, or 99% sequence identity to SEQ ID NO: 6. In some embodiments, the immunogenic fragment of hemagglutinin HA of influenza B Victoria in the mRNA molecule disclosed herein includes an amino acid sequence as set forth in SEQ ID NO: 10 or an amino acid sequence having at least 80%, 90%, 95%, or 99% sequence identity to SEQ ID NO: 10.

In some embodiments, the chimeric immunogenic polypeptide disclosed herein further includes an N-terminal leader sequence, the leader sequence preferably includes an amino acid sequence as set forth in SEQ ID NO: 2 or encoded by SEQ ID NO: 1, or an amino acid sequence having at least 80%, 90%, 95%, or 99% sequence identity to a sequence set forth in SEQ ID NO: 2 or encoded by SEQ ID NO: 1, and is capable of promoting proper folding of the chimeric immunogenic polypeptide.

In some embodiments, the mRNA disclosed herein includes a nucleotide sequence as set forth in SEQ ID NO: 11 or a nucleotide sequence having at least 80%, 90%, 95%, or 99% sequence identity to SEQ ID NO: 11. In some embodiments, the chimeric immunogenic polypeptide encoded by the mRNA molecule disclosed herein includes an amino acid sequence as set forth in SEQ ID NO: 12 or an amino acid sequence having at least 80%, 90%, 95%, or 99% sequence identity to SEQ ID NO: 12.

In some embodiments, the mRNA molecule disclosed herein further includes regulatory elements such as a 5' UTR, 3' UTR, and poly A tail operably linked to a polynucleotide sequence encoding the chimeric immunogenic polypeptide. In a preferred embodiment, the 5' UTR includes a sequence as set forth in SEQ ID NO: 23 or a sequence having at least 80%, 90%, 95%, 99% sequence identity to SEQ ID NO: 23, the 3' UTR includes a sequence as set forth in SEQ ID NO: 24 or a sequence having at least 80%, 90%, 95%, 99% sequence identity to SEQ ID NO: 24, and the poly A tail includes a sequence as set forth in SEQ ID NO: 25 or a sequence having at least 80%, 90%, 95%, 99% sequence identity to SEQ ID NO: 25.

In some embodiments, the mRNA molecule disclosed herein further includes a 5' cap. In a preferred embodiment, the 5' cap is m7G(5')ppp(5')(2'-OMeA)pG.

In some embodiments, the mRNA molecule disclosed herein further includes a chemical modification, for example, modification of all or part of uridylic acids in the polynucleotide sequence to 1-methylpseudouridine.

Another objective of the present invention is to provide a composition including the isolated mRNA molecule disclosed herein.

In some embodiments, the composition disclosed herein further includes an mRNA encoding an immunogenic fragment of hemagglutinin HA of another influenza A virus subtype and/or an mRNA encoding an immunogenic fragment of hemagglutinin HA of another influenza B virus subtype. In a preferred embodiment, the other influenza A virus subtypes are H3N2 and/or H7N9 subtypes, and the other influenza B virus subtype is a B/Yamagata subtype.

In some embodiments, the composition of the present invention further includes an mRNA encoding an immunogenic fragment of influenza virus matrix protein M1, influenza virus ion channel protein M2, and/or influenza virus nucleoprotein NP.

In some embodiments, the composition disclosed herein includes: a first mRNA including the nucleotide sequence encoding the chimeric immunogenic polypeptide disclosed herein; a second mRNA encoding a second immunogenic polypeptide, where the second immunogenic polypeptide includes an immunogenic fragment of H3N2 hemagglutinin HA; a third mRNA encoding a third immunogenic polypeptide, where the third immunogenic polypeptide includes an immunogenic fragment of H7N9 hemagglutinin HA; and a fourth mRNA encoding a fourth immunogenic polypeptide, where the fourth immunogenic polypeptide includes an immunogenic fragment of B/Yamagata hemagglutinin HA. In a preferred embodiment, a content ratio by mass of the first mRNA, the second mRNA, the third mRNA, and the fourth mRNA included in the composition of the present invention is in a range of (2-5):(2-5):(2-5):(0.5-5). In a more preferred embodiment, the content ratio by mass of the first mRNA, the second mRNA, the third mRNA, and the fourth mRNA included in the composition of the present invention is 5:5:2:0.5, 5:5:2:2.5, 5:2:2:0.5, or 5:5:5:5.

In some embodiments, the composition disclosed herein includes: a first mRNA including the nucleotide sequence encoding the chimeric immunogenic polypeptide disclosed herein; a second mRNA encoding a second immunogenic polypeptide, where the second immunogenic polypeptide includes the immunogenic fragment of H3N2 hemagglutinin HA; a third mRNA encoding a third immunogenic polypeptide, where the third immunogenic polypeptide includes the immunogenic fragment of H7N9 hemagglutinin HA; a fourth mRNA encoding a fourth immunogenic polypeptide, where the fourth immunogenic polypeptide includes the immunogenic fragment of B/Yamagata hemagglutinin HA; and a fifth mRNA encoding a fifth immunogenic polypeptide, where the fifth immunogenic polypeptide includes the immunogenic fragment of influenza virus ion channel protein M2. In a preferred embodiment, a content ratio by mass of the first mRNA, the second mRNA, the third mRNA, the fourth mRNA, and the fifth mRNA included in the composition disclosed herein is in a range of (2-5):(2-5):(2-5):(0.5-5):(2-5). In a more preferred embodiment, the content ratio by mass of the first mRNA, the second mRNA, the third mRNA, the fourth mRNA, and the fifth mRNA included in the composition disclosed herein is 5:5:5:5:5, 5:5:2:2:2, 5:2:2:2:2, 5:2:2:0.5:0.5, 5:5:2:0.5:2.5, or 5:2:2:0.5:3.

In some embodiments, the hemagglutinin antigen in the composition disclosed herein is recommended or selected according to standardized criteria used by the World Health Organization's Global Influenza Surveillance and Response System (GISRS).

In some embodiments, the chimeric immunogenic polypeptide described herein includes an amino acid sequence as set forth in SEQ ID NO: 12 or an amino acid sequence having at least 80%, 90%, 95%, or 99% sequence identity to SEQ ID NO: 12.

In some embodiments, a sequence of the immunogenic fragment of hemagglutinin HA of H3N2 described herein includes an amino acid sequence set forth in SEQ ID NO: 14 or a sequence having at least 80%, 90%, 95%, or 99% sequence identity to SEQ ID NO: 14.

In some embodiments, a sequence of the immunogenic fragment of hemagglutinin HA of H7N9 described herein includes an amino acid sequence set forth in SEQ ID NO: 16 or a sequence having at least 80%, 90%, 95%, or 99% sequence identity to SEQ ID NO: 16.

In some embodiments, a sequence of the immunogenic fragment of hemagglutinin HA of B/Yamagata described herein includes an amino acid sequence set forth in SEQ ID NO: 18 or a sequence having at least 80%, 90%, 95%, or 99% sequence identity to SEQ ID NO: 18.

In some embodiments, a sequence of the immunogenic fragment of influenza virus ion channel protein M2 described herein includes an amino acid sequence set forth in SEQ ID NO: 20 or a sequence having at least 80%, 90%, 95%, or 99% sequence identity to SEQ ID NO: 20.

In some embodiments, the first mRNA described herein includes a nucleotide sequence as set forth in SEQ ID NO: 11 or a sequence having at least 80%, 90%, 95%, or 99% sequence identity to SEQ ID NO: 11.

In some embodiments, the second mRNA described herein includes a nucleotide sequence as set forth in SEQ ID NO: 13 or a sequence having at least 80%, 90%, 95%, or 99% sequence identity to SEQ ID NO: 13.

In some embodiments, the third mRNA described herein includes a nucleotide sequence as set forth in SEQ ID NO: 15 or a sequence having at least 80%, 90%, 95%, or 99% sequence identity to SEQ ID NO: 15.

In some embodiments, the fourth mRNA described herein includes a nucleotide sequence as set forth in SEQ ID NO: 17 or a sequence having at least 80%, 90%, 95%, or 99% sequence identity to SEQ ID NO: 17.

In some embodiments, the fifth mRNA described herein includes a nucleotide sequence as set forth in SEQ ID NO: 19 or a sequence having at least 80%, 90%, 95%, or 99% sequence identity to SEQ ID NO: 19.

In some embodiments, the composition disclosed herein includes (1) the nucleotide sequence encoding the chimeric immunogenic polypeptide, where the chimeric immunogenic polypeptide includes an amino acid sequence as set forth in SEQ ID NO: 12 or an amino acid sequence having at least 80%, 90%, 95%, or 99% sequence identity to SEQ ID NO: 12, (2) a nucleotide sequence encoding the immunogenic fragment sequence of hemagglutinin HA of H3N2, where the immunogenic fragment sequence of hemagglutinin HA of H3N2 includes an amino acid sequence set forth in SEQ ID NO: 14 or a sequence having at least 80%, 90%, 95%, or 99% sequence identity to SEQ ID NO: 14, (3) a nucleotide sequence encoding the immunogenic fragment sequence of hemagglutinin HA of H7N9, where the immunogenic fragment sequence of hemagglutinin HA of H7N9 includes an amino acid sequence set forth in SEQ ID NO: 16 or a sequence having at least 80%, 90%, 95%, or 99% sequence identity to SEQ ID NO: 16, and (4) a nucleotide sequence encoding the immunogenic fragment sequence of hemagglutinin HA of B/Yamagata, where the immunogenic fragment sequence of hemagglutinin HA of B/Yamagata includes an amino acid sequence set forth in SEQ ID NO: 18 or a sequence having at least 80%, 90%, 95%, or 99% sequence identity to SEQ ID NO: 18.

In some embodiments, the composition disclosed herein includes (1) the nucleotide sequence encoding the chimeric immunogenic polypeptide, where the chimeric immunogenic polypeptide includes an amino acid sequence as set forth in SEQ ID NO: 12 or an amino acid sequence having at least 80%, 90%, 95%, or 99% sequence identity to SEQ ID NO: 12, (2) a nucleotide sequence encoding the immunogenic fragment sequence of hemagglutinin HA of H3N2, where the immunogenic fragment sequence of hemagglutinin HA of H3N2 includes an amino acid sequence set forth in SEQ ID NO: 14 or a sequence having at least 80%, 90%, 95%, or 99% sequence identity to SEQ ID NO: 14, (3) a nucleotide sequence encoding the immunogenic fragment sequence of hemagglutinin HA of H7N9, where the immunogenic fragment sequence of hemagglutinin HA of H7N9 includes an amino acid sequence set forth in SEQ ID NO: 16 or a sequence having at least 80%, 90%, 95%, or 99% sequence identity to SEQ ID NO: 16, (4) a nucleotide sequence encoding the immunogenic fragment sequence of hemagglutinin HA of B/Yamagata, where the immunogenic fragment sequence of hemagglutinin HA of B/Yamagata includes an amino acid sequence set forth in SEQ ID NO: 18 or a sequence having at least 80%, 90%, 95%, or 99% sequence identity to SEQ ID NO: 18, and (5) a nucleotide sequence encoding an immunogenic fragment sequence of influenza virus ion channel protein M2, where the immunogenic fragment sequence of influenza virus ion channel protein M2 includes an amino acid sequence set forth in SEQ ID NO: 20 or a sequence having at least 80%, 90%, 95%, or 99% sequence identity to SEQ ID NO: 20.

In some embodiments, the composition disclosed herein includes: (1) a first mRNA including a nucleotide sequence as set forth in SEQ ID NO: 11 or a sequence having at least 80%, 90%, 95%, or 99% sequence identity to SEQ ID NO: 11; (2) a second mRNA including a nucleotide sequence as set forth in SEQ ID NO: 13 or a sequence having at least 80%, 90%, 95%, or 99% sequence identity to SEQ ID NO: 13; (3) a third mRNA including a nucleotide sequence as set forth in SEQ ID NO: 15 or a sequence having at least 80%, 90%, 95%, or 99% sequence identity to SEQ ID NO: 15; and (4) a fourth mRNA including a nucleotide sequence as set forth in SEQ ID NO: 17 or a sequence having at least 80%, 90%, 95%, or 99% sequence identity to SEQ ID NO: 17.

In some embodiments, the composition disclosed herein includes: (1) a first mRNA including a nucleotide sequence as set forth in SEQ ID NO: 11 or a sequence having at least 80%, 90%, 95%, or 99% sequence identity to SEQ ID NO: 11; (2) a second mRNA including a nucleotide sequence as set forth in SEQ ID NO: 13 or a sequence having at least 80%, 90%, 95%, or 99% sequence identity to SEQ ID NO: 13; (3) a third mRNA including a nucleotide sequence as set forth in SEQ ID NO: 15 or a sequence having at least 80%, 90%, 95%, or 99% sequence identity to SEQ ID NO: 15; (4) a fourth mRNA including a nucleotide sequence as set forth in SEQ ID NO: 17 or a sequence having at least 80%, 90%, 95%, or 99% sequence identity to SEQ ID NO: 17; and (5) a fifth mRNA including a nucleotide sequence as set forth in SEQ ID NO: 19 or a sequence having at least 80%, 90%, 95%, or 99% sequence identity to SEQ ID NO: 19.

In some embodiments, the mRNA molecules in the composition disclosed herein further include regulatory elements such as a 5' UTR, 3' UTR, and poly A tail operably linked to the encoding nucleotide sequence. In a preferred embodiment, the 5' UTR includes a sequence as set forth in SEQ ID NO: 23 or a sequence having at least 80%, 90%, 95%, 99% sequence identity to SEQ ID NO: 23, the 3' UTR includes a sequence as set forth in SEQ ID NO: 24 or a sequence having at least 80%, 90%, 95%, 99% sequence identity to SEQ ID NO: 24, and the poly A tail includes a sequence as set forth in SEQ ID NO: 25 or a sequence having at least 80%, 90%, 95%, 99% sequence identity to SEQ ID NO: 25.

In some embodiments, the mRNA molecule in the composition disclosed herein further includes a 5' cap, and preferably the included 5' cap is m7G(5')ppp(5')(2'-OMeA)pG.

In some embodiments, the mRNA molecule in the composition disclosed herein further includes a chemical modification, for example, modification of all or part of uridylic acids in the nucleotide sequence to 1-methylpseudouridine.

In some embodiments, the composition disclosed herein further includes a pharmaceutically acceptable carrier. In a preferred embodiment, the carrier is a lipid nanoparticle, and the mRNA disclosed herein is encapsulated in one or more lipid nanoparticles.

In some embodiments, each individual particle in the lipid nanoparticles encapsulates one or more of the first to fifth mRNAs in substantially the same ratio. In other embodiments, individual particles in the lipid nanoparticles may encapsulate one or more of the first to fifth mRNAs in different ratios. In other embodiments, individual particles in the lipid nanoparticles separately encapsulate any one of the first, second, third, fourth, or fifth mRNA.

In some embodiments, each individual particle in the lipid nanoparticles encapsulates the first to fourth mRNAs in substantially the same ratio. In other embodiments, individual particles in the lipid nanoparticles may encapsulate the first to fourth mRNAs in different ratios. In still other embodiments, individual particles in the lipid nanoparticles separately encapsulate any one of the first, second, third, or fourth mRNA.

In some embodiments, each individual particle in the lipid nanoparticles encapsulates the first to fifth mRNAs in substantially the same ratio. In other embodiments, individual particles in the lipid nanoparticles may encapsulate the first to fifth mRNAs in different ratios. In still other embodiments, individual particles in the lipid nanoparticles separately encapsulate any one of the first, second, third, fourth, or fifth mRNA.

In some embodiments, the lipid nanoparticles include an ionizable lipid, a phospholipid, a structural lipid, and a polyethylene glycol (PEG)-lipid. In a preferred embodiment, a molar ratio of the ionizable lipid, a sum of the phospholipid and the structural lipid, and the PEG-lipid in the lipid nanoparticles is (40-65):(35-65):(1-3). In a more preferred embodiment, the lipid nanoparticles include the ionizable lipid, the phospholipid, the structural lipid, and the PEG-lipid at a molar ratio of (40-55):(5-15):(30-50):(1-3).

In some embodiments, the lipid nanoparticles include an ionizable lipid, a phospholipid, a structural lipid, and a polyethylene glycol (PEG)-lipid. In a preferred embodiment, the lipid nanoparticles include the ionizable lipid, the phospholipid, the structural lipid, and the PEG-lipid at a molar ratio of (20-60):(5-25):(25-55):(0.5-5).

In some embodiments, the lipid nanoparticles include one or two or more selected from the following phospholipid compounds: dilauroyl phosphatidylcholine (DLPC), dimyristoyl phosphatidylcholine (DMPC), dioleoyl phosphatidylcholine (DOPC), dipalmitoyl phosphatidylcholine (DPPC), distearoyl phosphatidylcholine (DSPC), dioleoyl phosphatidylcholine (DUPC), palmitoyloleoyl phosphatidylcholine (POPC), 1,2-di-O-octadecyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-divinyl-sn-glycero-3-phosphocholine, 1,2-diarachidoyl-sn-glycero-3-phosphocholine, 1,2-dioleoyl-sn-glycero-3-phosphoethanolamine (DOPE), 1,2-distearoyl-sn-glycero-3-phosphoethanolamine, 1,2-bis(hydroxyvinyl)-sn-glycero-3-phosphoethanolamine, 1,2-divinyl-sn-glycero-3-phosphoethanolamine, 1,2-diarachidoyl-sn-glycero-3-phosphoethanolamine, 1,2-dithiohexaenoic acid-sn-glycero-3-phosphoethanolamine, 1,2-dioleoyl-sn-glycero-3-phospho-(1'-glycerol) sodium salt (DOPG), or sphingomyelin. In a preferred embodiment, the lipid nanoparticles include DSPC.

In some embodiments, the lipid nanoparticles include one or two or more selected from the following structural lipids: cholesterol, coprostanol, sitosterol, ergosterol, stigmasterol. In a preferred embodiment, the structural lipid is cholesterol.

In some embodiments, the lipid nanoparticles include one or two or more selected from the following PEG-lipids: PEG-modified phosphatidylethanolamine, PEG-modified phosphatidic acid, PEG-modified ceramide, PEG-modified dialkylamine, PEG-modified diacylglycerol, or PEG-modified dialkylglycerol. In a preferred embodiment, the lipid nanoparticles include DMG-PEG2000.

In some embodiments, the composition disclosed herein is a vaccine.

Another objective of the present invention is to provide a nucleic acid molecule encoding the mRNA disclosed herein, where the nucleic acid molecule is preferably a DNA, more preferably a DNA plasmid.

Another objective of the present invention is to provide a fusion protein, including an amino acid sequence encoded by the first mRNA disclosed herein, which is useful for preventing or treating influenza virus infection. Further provided is a composition including the fusion protein disclosed herein.

Another objective of the present invention is to provide a method for inducing an immune response against an influenza virus in a subject, including administering to the subject an effective dose of the isolated mRNA, the composition, the vector, or the fusion protein disclosed herein. In a preferred embodiment, the method disclosed herein includes administering to the subject two or three effective doses of the isolated mRNA, the composition, the nucleic acid molecule, or the fusion protein disclosed herein.

Another objective of the present invention is to provide use of the isolated mRNA, the composition, the nucleic acid molecule, or the fusion protein disclosed herein in preparation of a medicament for preventing or treating influenza virus infection.

### BRIEF DESCRIPTION OF THE DRAWINGS

FIG. 1 shows results of hemagglutination inhibition tests after mice are immunized with mRNA-LNP compositions of groups G1, G3, and G4 prepared in Example 2 (n.s.: not significant, *: p < 0.05, using Multiple t-test);
FIG. 2 shows results of hemagglutination inhibition tests after mice are immunized with mRNA-LNP compositions of groups G1, G2, and G5 prepared in Example 2; and
FIG. 3 shows IgG titers against H1N1, H7N9, H5N1, H3N2, B/Victoria, and B/Yamagata simultaneously detectable in mouse sera when mice are immunized with mRNA-LNP compositions of groups G1 and G6 prepared in Example 2.

### DETAILED DESCRIPTION

The present invention will be described in further detail below in conjunction with specific embodiments. The examples provided are intended only to illustrate the present invention, rather than to limit the scope of the present invention. The examples provided below may serve as a guide for further improvement by those of ordinary skill in the art, and do not constitute any limitation to the present invention in any manner.

Unless otherwise specified, the experimental methods in the following examples are conventional methods, and performed according to the techniques or conditions described in literature in the art or according to product instructions. Specific references may be made to, for example, Sambrook et al., Molecular Cloning: a Laboratory Manual, 4th Edition, Cold Spring Harbor Laboratory Press, 2012; Ausubel et al., Current Protocols in Molecular Biology, Wiley Online Press, updated periodically. Unless otherwise specified, materials, reagents, instruments, etc., used in the following examples are commercially available. For quantitative tests in the following examples, data are expressed as the mean of three replicate experiments, unless otherwise specified. In the following examples, unless otherwise specified, each nucleotide sequence in the sequence listing is written from left to right in the order from the 5' end to the 3' end, and each amino acid sequence is written from left to right in the order from the amino terminus to the carboxyl terminus.

### Definitions

Unless otherwise defined herein, scientific and technical terms used in connection with the present disclosure shall have the meanings that are commonly understood by those of ordinary skill in the art. The meaning and scope of the terms should be clear; however, in the event of any potential ambiguity, the definitions provided herein take precedence over any dictionary or external definitions.

As used herein, the term "comprise" or "include" means that a sequence, a composition, and a method include the recited components or steps, but do not exclude other components or steps. "Consisting essentially of," when used to define a composition and a method, shall mean excluding other components or steps that are materially important to the technical effect that they are intended to achieve. "Consisting of" shall mean excluding other components and steps not mentioned.

Unless the context clearly indicates otherwise, the singular forms "a," "an," and "the" include plural referents. Thus, for example, reference to "a cell" includes a combination of two or more cells, or the entire culture of cells. As used herein, the term "or" is understood to be inclusive, unless explicitly stated or obvious from the context.

As used herein, the term "about" should be understood to be within the normal tolerance of the art, e.g., within 2 standard deviations of the mean, unless explicitly stated or obvious from the context. "About" can be understood as within 10%, 9%, 8%, 7%, 6%, 5%, 4%, 3%, 2%, 1%, 0.9%, 0.8%, 0.7%, 0.6%, 0.5%, 0.4%, 0.3%, 0.2%, 0.1%, 0.09%, 0.08%, 0.07%, 0.06%, 0.05%, 0.04%, 0.03%, 0.02%, or 0.01% of the stated value. All numerical values provided herein are modified by the term "about," unless otherwise obvious from the context.

As used herein, the term "isolated" means that a biological component (such as a nucleic acid) has been substantially separated or purified from other biological components (such as other chromosomal and extrachromosomal DNAs and RNAs, proteins, and organelles) in the environment (e.g., a cell) in which the component naturally occurs. An "isolated" nucleic acid includes a nucleic acid purified by standard purification methods. The term also includes a nucleic acid prepared by recombinant expression in a host cell as well as a chemically synthesized nucleic acid.

As used herein, the term "immunogenic polypeptide" refers to a protein (including a glycoprotein) and a peptide capable of eliciting an immune response in a mammal, including an immunologically active peptide that, upon administration to a host, can elicit a humoral and/or cellular immune response against the polypeptide and/or against a homologous polypeptide having an amino acid sequence with high identity (e.g., at least 60%, 65%, 70%, 75%, 80%, 85%, 87%, 90%, 92%, 93%, 94%, 95%, 96%, 97%, 98%, or 99% identity) to the particular polypeptide. Accordingly, an "immunogenic polypeptide" herein includes a full-length sequence of a polypeptide, an analog thereof, or an immunogenic fragment thereof. The term "immunogenic fragment" as used herein may refer to a polypeptide fragment of at least one selected from the following amino acid sequences: a protein/polypeptide fragment that includes, consists essentially of, or consists of at least one epitope or antigenic determinant, thereby being capable of eliciting an immune response. As used herein, an "immunogenic fragment" may include deletions, substitutions, and/or additions relative to the polypeptide sequence from which it is derived, provided that the fragment functions to elicit an immune response against the polypeptide.

In some embodiments, the immunogenic polypeptide is a full-length, fragment, derivative, or variant of an influenza virus hemagglutinin HA antigen. For example, in some embodiments, the HA is a wild-type HA. In other embodiments, the HA is a modified HA, e.g., an HA including at least one amino acid substitution, deletion, and/or insertion such that its primary structure differs from that of the wild-type HA. In some specific embodiments, the mutations in HA are T219I, H371Y, I494M, H504P, M362L, HA0, APB, TB, or VASP, or any combination thereof. In some embodiments, the mutations in HA are the formation of a disulfide bond in the HA stem to link adjacent protomers, deletion of a cleavage site, and/or replacement of a polybasic cleavage site (HPAI) with an LPAI sequence.

Influenza antigens may be recommended or selected according to the standardized criteria used by the World Health Organization's Global Influenza Surveillance and Response System (GISRS). In some embodiments, the HA antigens and NA antigens recommended or selected by GISRS are included in influenza virus vaccines of the southern hemisphere or northern hemisphere influenza vaccines for the year of manufacture and distribution. In some embodiments, hemagglutination inhibition (HAI) assays are used to select HA antigens and NA antigens to identify circulating influenza viruses that are antigenically similar to the influenza viruses of the previous season's vaccine, optionally where influenza viruses are considered antigenically similar if their HAI titers differ by two-fold or less.

The term "chimeric" has its ordinary meaning as understood in the art, referring to the linkage of at least two building blocks of different origins (such as amino acid sequences or nucleotide sequences) to form a new entity (such as a new polypeptide or nucleic acid molecule). When describing the immunogenic polypeptide herein, "chimeric" means that the immunogenic polypeptide referred to includes at least two epitopes or antigenic determinants from different antigens, or at least two epitopes or antigenic determinants from the same antigen but linked together in an altered connection order.

As used herein, a "linker" refers to a peptide sequence or a non-peptide structure that connects two polypeptide fragments to each other. In some embodiments, the linker is a flexible peptide linker that allows a certain mobility of the two amino acid fragments being linked. Addition of Ser and Thr may allow the linker to form hydrogen bonds with water molecules, conferring stability to the linker in aqueous solution, thereby reducing interactions between the linker and the preceding and succeeding proteins. Common flexible peptide linkers consist of Gly and Ser residues ("GS" linker). In addition to GS linkers, there are other flexible linkers, such as (Gly)₈ and the like, all of which are known in the art. In some embodiments, the linker is a rigid linker, which can be used to completely separate two linked proteins and maintain their independent functions. Commonly used rigid linkers include α-helical structure peptides, (XP)ₙ, and the like, where P represents proline, X may be any amino acid, preferably Ala, Lys, Glu, and n represents the number of XP repeats. Those skilled in the art can independently adjust and select different linkers according to specific application scenarios and the 3D structure requirements of the fusion protein.

As used herein, the "head" and "stem" of hemagglutinin (HA) refer to the globular head domain and stem domain (or stalk domain) of the hemagglutinin protein, respectively. The corresponding hemagglutinin (HA) fragments are known to those skilled in the art (e.g., obtainable from public databases such as GenBank) or can be determined according to the method described, for example, in the literature "Influenza Viruses Expressing Chimeric Hemagglutinins: Globular Head and Stalk Domains Derived from Different Subtypes doi:10.1128/JVI.00137-12."

As used herein, the term "identity" refers to the percentage of identical residues possessed by two or more nucleic acid or polypeptide sequences over a specified region. Methods for determining the percent identity between two amino acid sequences or nucleotide sequences are well known in the art. For example, the identity between two sequences can be calculated as follows: the two sequences are optimally aligned over a specified region, the number of positions at which identical residues appear in both sequences is determined as the number of matched positions, the number of matched positions is divided by the total number of positions in the specified region, and the result is multiplied by 100. In cases where the two sequences differ in length, or the alignment produces one or more staggered ends, and a particular comparison region includes only a single sequence, residues of the single sequence are included in the denominator but not the numerator of the calculation. When comparing a DNA and an RNA, thymine (T) and uracil (U) may be considered equivalent. Identity can be determined manually or using computer sequence algorithms (such as BLAST or BLAST 2.0).

As used herein, the term "5' cap" is located at the 5' most end of mRNA and includes a methylated guanylate linked via a pyrophosphate to the 5'-end of mRNA, forming a 5',5'-triphosphate linkage with the adjacent nucleotide. There are generally three types of 5' cap structures (m7G5'ppp5'Np, m7G5'ppp5'NmpNp, m7G5'ppp5'NmpNmpNp), referred to as Type O, Type I, and Type II, respectively. Type O means the ribose of the terminal nucleotide is unmethylated, Type I means the ribose of one terminal nucleotide is methylated, and Type II means the riboses of two terminal nucleotides are methylated. In the present disclosure, a preferred 5' cap is m7G(5')ppp(5')(2'-OMeA)pG (commercially available from, e.g., TriLink Biotechnologies).

As used herein, the terms "PolyA tail" or "PolyA sequence" refer to a sequence of uninterrupted or interrupted adenosine residues typically located at the 3'-end of an RNA molecule. Poly-A tails or Poly-A sequences are known to those skilled in the art and can be selected according to actual needs. In mRNA, where a 3'-UTR is present, the Poly-A sequence is attached to the 3' end of the 3'-UTR. An uninterrupted poly-A tail is characterized by consecutive adenosine residues. The Poly-A tail may be of any length. In some embodiments, the Poly-A tail includes or consists of at least 20, at least 30, at least 40, at least 80, or at least 100, and at most 500, at most 400, at most 300, at most 200, or at most 150 adenosines (A), particularly about 120 A's. Typically, the vast majority of nucleotides in the PolyA tail are adenosines, where the vast majority means at least 75%, at least 80%, at least 85%, at least 90%, at least 95%, at least 96%, at least 97%, at least 98%, or at least 99% of the nucleotides, while the remaining nucleotides are allowed to be nucleotides other than A, such as U (uridylic acid), G (guanylic acid), or C (cytidylic acid).

As used herein, the terms "individual," "subject," "patient," "host," "subject in need thereof," or similar expressions refer to any mammal or non-mammal. Mammals include, but are not limited to, cats, and other vertebrates such as rodents, humans, non-human primates, for example, cattle, horses, dogs, pigs, sheep, goats, giraffes, deer, camels, antelopes, rats, mice, hares, and rabbits.

In the vaccination methods of this application, the subject to be vaccinated may have been exposed to an influenza virus. As used herein, the terms "exposed," "exposure," and the like indicate that the subject has come into contact with a human or animal known to be infected with an influenza virus. The vaccine of this application may be administered using techniques well known to those skilled in the art. For formulation and administration techniques, reference may be made to, for example, *Remington's Pharmaceutical Sciences*, 18th edition. The vaccine may be administered by means including, but not limited to, traditional syringes, needle-free injection devices, or microprojectile bombardment gene guns. Suitable routes of administration include, but are not limited to, parenteral administration such as intramuscular, intradermal, subcutaneous, or intramedullary injection, as well as intrathecal, direct intraventricular, intravenous, intraperitoneal, intranasal, or intraocular injection. For injection, the nanoparticles of this application may be formulated in an aqueous solution, preferably in a physiologically compatible buffer such as Hank's solution, Ringer's solution, or normal saline buffer.

As used herein, the term "lipid nanoparticle" (LNP) refers to a particle having at least one dimension on the nanometer scale and including at least one lipid. In a preferred embodiment, the lipid includes, but is not limited to, a neutral phospholipid and a polyethylene glycol-lipid. As used herein, the term "neutral phospholipid" refers to an uncharged, non-phosphoglyceride phospholipid molecule. As used herein, the term "polyethylene glycol-lipid" refers to a molecule including a lipid moiety and a polyethylene glycol moiety.

The lipid nanoparticles generally include an ionizable lipid, a non-cationic lipid, a sterol, a PEG-lipid component, and a target nucleic acid such as an mRNA. The lipid nanoparticles of the present disclosure can be produced using components, compositions, and methods as generally known in the art, see, for example, PCT/US2016/052352, PCT/US2016/068300, PCT/US2017/037551, PCT/US2015/027400, PCT/US2016/047406, PCT/US2016/000129, PCT/US2016/014280, PCT/US2016/014280, PCT/US2017/038426, PCT/US2014/027077, PCT/US2014/055394, PCT/US2016/52117, PCT/US2012/069610, PCT/US2017/027492, PCT/US2016/059575, and PCT/US2016/069491, all of which are incorporated herein by reference in their entirety.

As used herein, a "neutralizing antibody" refers to an antibody that prevents an influenza virus from completing one round of replication. One round of replication refers to the life cycle of the virus, beginning with viral attachment to a host cell and ending with budding of a newly formed virus from the host cell. This life cycle includes, but is not limited to, the steps of attachment to a cell, internalization, uncoating and rearrangement, fusion of the viral membrane with the endosomal membrane, release of viral ribonucleoproteins into the cytoplasm, formation of new viral particles, and budding of viral particles from the host cell membrane. A neutralizing antibody is an antibody that inhibits one or more of the above steps. A "broadly neutralizing antibody" is an antibody that neutralizes influenza viruses of one or more types, groups, subtypes, and/or strains. For example, a broadly neutralizing antibody induced against the HA protein of an influenza A virus may neutralize an influenza B or influenza C virus.

As used herein, an "influenza virus" refers to any influenza virus strain capable of causing a disease in an animal or human subject. See, for example, Fields, B. et al., Fields' Virology, 4th Edition, Philadelphia: Lippincott Williams and Wilkins; ISBN: 0781718325, 2001. Specifically, the term primarily includes any strain of an influenza A virus (or type A influenza virus) and an influenza B virus (or type B influenza virus) capable of causing a disease in an animal or human subject. The influenza A virus includes different subtypes, typically defined by the types of hemagglutinin (HA) and/or neuraminidase (NA) of the virus. It is known in the art that there are 18 distinct HA subtypes (H1 to H18) and 11 distinct NA subtypes (N1 to N11); see, for example, CDC, Types of Influenza Viruses, 2019. Non-limiting examples of influenza A virus strains include, for example, H1N1, H5N1, H3N2, H6N2, H7N3, H7N7, H9N2, H10N4, and H10N5. These include, but are not limited to: A/Puerto Rico/8/34, A/Victoria/4897/2022, A/Wisconsin/67/2022, A/Victoria/2570/2019, A/Sydney/5/2021, A/California/07/2009, A/Michigan/45/2015, A/Brisbane/02/2018, A/Hawaii/70/2019, A/Idaho/07/2018, A/Maine/38/2018, A/Nebraska/15/2018, A/Nebraska/14/2019, A/Wisconsin/588/2019, A/Iowa/33/2019, A/Arkansas/28/2019, A/Virginia/41/2019, A/Minnesota/60/2019, A/Alabama/27/2019, or A/Guangdong-Maonan/SWL1536/2019; A/Aichi/2/1968, A/Thailand/8/2022, A/Massachusetts/18/2022, A/Darwin/9/2021, A/Darwin/6/2021, A/Cambodia/e0826360/2020, A/Switzerland/8060/2017, A/Switzerland/9715293/2013, A/Iowa/60/2018, A/South Australia/34/2019, A/Hong Kong/45/2019, A/Hong Kong/2671/2019, A/Kansas/14/2017, A/Jamaica/60361/2019, A/Florida/130/2019, A/Laos/1789/2019, A/Vermont/25/2019, A/New Jersey/34/2019, A/California/176/2019, A/Pennsylvania/1026/2019, A/Togo/634/2019, A/Kenya/130/2019, A/Togo/1307/2019, A/Ohio/30/2019, A/Guatemala/93/2019, A/Guatemala/10/2019, A/Hong Kong/4801/2014, or A/Singapore/INFIMH-16-0019/2016.

The influenza B virus includes any influenza B virus strain known in the art, examples of which include, but are not limited to, influenza B virus strains derived from the following locations: Aichi, Akita, Alaska, Ann Arbor, Argentina, Bangkok, Beijing, Belgium, Bonn, Brazil, Buenos Aires, Canada, Chaco, Chiba, Chongqing, CNIC, Cordoba, Czechoslovakia, Daegu, Durban, Finland, Fujian, Fukuoka, Genoa, Guangdong, Guangzhou, Hannover, Harbin, Hawaii, Hebei, Henan, Hiroshima, Hong Kong, Houston, Hunan, Ibaraki, India, Israel, Johannesburg, Kagoshima, Kanagawa, Kansas, Khazkov, Kobe, Kochi, Lazio, Lee, Leningrad, Lisbon, Los Angeles, Lusaka, Lyon, Malaysia, Maputo, Mar del Plata, Maryland, Memphis, Michigan, Mie, Milan, Minsk, Nagasaki, Nagoya, Nanchang, Nashville, Nebraska, Netherlands, New York, NIB, Ningxia, Norway, Oman, Oregon, Osaka, Oslo, Panama, Paris, Parma, Perugia, Philippines, Busan, Quebec, Rochester, Rome, Saga, Seoul, Shandong, Shanghai, Shenzhen, Shiga, Shizuoka, Sichuan, Siena, Singapore, South Carolina, South Dakota, Spain, Stockholm, Switzerland, Taiwan Province of China, Texas, Tokushima, Tokyo, Trento, Trieste, United Kingdom, Ushuaia, USSR, Utah, Victoria, Vienna, Wuhan, Xuanwu, Yamagata, Yamanashi, Yunnan, as well as hybrid subtypes, circulating recombinant forms, clinical and field isolates thereof. Exemplary influenza B virus strains include, but are not limited to: an Akita/27/2001 strain, an Akita/5/2001 strain, an Alaska/16/2000 strain, an Alaska/1777/2005 strain, an Argentina/69/2001 strain, an Arizona/146/2005 strain, an Arizona/148/2005 strain, a Bangkok/163/90 strain, a Bangkok/34/99 strain, a Bangkok/460/03 strain, a Bangkok/54/99 strain, a Barcelona/215/03 strain, a Beijing/15/84 strain, a Beijing/184/93 strain, a Beijing/243/97 strain, a Beijing/43/75 strain, a Beijing/5/76 strain, a Beijing/76/98 strain, a Belgium/WV 106/2002 strain, a Belgium/WV 107/2002 strain, a Belgium/WV 109/2002 strain, a Belgium/WV114/2002 strain, a Belgium/WV122/2002 strain, a Bonn/43 strain, a Brazil/952/2001 strain, a Brisbane/60/2008 (B/Victoria) strain, a Bucharest/795/03 strain, a Buenos Aires/161/00 strain, a Buenos Aires/9/95 strain, a Buenos Aires/SW16/97 strain, a Buenos Aires/VL518/99 strain, a Canada/464/2001 strain, a Canada/464/2002 strain, a Chaco/366/00 strain, a Chaco/R113/00 strain, a Cheju/303/03 strain, a Chiba/447/98 strain, a Chongqing/3/2000 strain, a clinical isolate SA1 Thailand/2002, a clinical isolate SA10 Thailand/2002, a clinical isolate SA100 Philippines/2002, a clinical isolate SA101 Philippines/2002, a clinical isolate SA110 Philippines/2002, a clinical isolate SA112 Philippines/2002, a clinical isolate SA113 Philippines/2002, a clinical isolate SA114 Philippines/2002, a clinical isolate SA2 Thailand/2002, a clinical isolate SA20 Thailand/2002, a clinical isolate SA38 Philippines/2002, a clinical isolate SA39 Thailand/2002, a clinical isolate SA99 Philippines/2002, a CNIC/27/2001 strain, a Colorado/2597/2004 strain, a Cordoba/VA418/99 strain, a Czechoslovakia/16/89 strain, a Czechoslovakia/69/90 strain, a Daegu/10/97 strain, a Daegu/45/97 strain, a Daegu/47/97 strain, a Daegu/9/97 strain, a B/Du/4/78 strain, a B/Durban/39/98 strain, a Durban/43/98 strain, a Durban/44/98 strain, a B/Durban/52/98 strain, a Durban/55/98 strain, a Durban/56/98 strain, an England/1716/2005 strain, an England/2054/2005 strain, an England/23/04 strain, a Finland/154/2002 strain, a Finland/159/2002 strain, a Finland/160/2002 strain, a Finland/161/2002 strain, a Finland/162/03 strain, a Finland/162/2002 strain, a Finland/162/91 strain, a Finland/164/2003 strain, a Finland/172/91 strain, a Finland/173/2003 strain, a Finland/176/2003 strain, a Finland/184/91 strain, a Finland/188/2003 strain, a Finland/190/2003 strain, a Finland/220/2003 strain, a Finland/WV5/2002 strain, a Fujian/36/82 strain, a Geneva/5079/03 strain, a Genoa/11/02 strain, a Genoa/2/02 strain, a Genoa/21/02 strain, a Genova/54/02 strain, a Genova/55/02 strain, a Guangdong/05/94 strain, a Guangdong/08/93 strain, a Guangdong/5/94 strain, a Guangdong/55/89 strain, a Guangdong/8/93 strain, a Guangzhou/7/97 strain, a Guangzhou/86/92 strain, a Guangzhou/87/92 strain, a Gyeonggi/592/2005 strain, a Hannover/2/90 strain, a Harbin/07/94 strain, a Hawaii/10/2001 strain, a Hawaii/1990/2004 strain, a Hawaii/38/2001 strain, a Hawaii/9/2001 strain, a Hebei/19/94 strain, a Hebei/3/94 strain, a Henan/22/97 strain, a Hiroshima/23/2001 strain, a Hong Kong/110/99 strain, a Hong Kong/1115/2002 strain, a Hong Kong/112/2001 strain, a Hong Kong/123/2001 strain, a Hong Kong/1351/2002 strain, a Hong Kong/1434/2002 strain, a Hong Kong/147/99 strain, a Hong Kong/156/99 strain, a Hong Kong/157/99 strain, a Hong Kong/22/2001 strain, a Hong Kong/22/89 strain, a Hong Kong/336/2001 strain, a Hong Kong/666/2001 strain, a Hong Kong/9/89 strain, a Houston/1/91 strain, a Houston/1/96 strain, a Houston/2/96 strain, a Hunan/4/72 strain, an Ibaraki/2/85 strain, an Incheon/297/2005 strain, an India/3/89 strain, an India/77276/2001 strain, an Israel/95/03 strain, an Israel/WV 187/2002 strain, a Japan/1224/2005 strain, a Jiangsu/10/03 strain, a Johannesburg/1/99 strain, a Johannesburg/96/01 strain, a Kadoma/1076/99 strain, a Kadoma/122/99 strain, a Kagoshima/15/94 strain, a Kansas/22992/99 strain, a Khazkov/224/91 strain, a Kobe/1/2002 strain, a Kochi/193/99 strain, a Lazio/1/02 strain, a Lee/40 strain, a Leningrad/129/91 strain, a Lissabon/2/90 strain, a Los Angeles/1/02 strain, a Lusaka/270/99 strain, a Lyon/1271/96 strain, a Malaysia/83077/2001 strain, a Maputo/1/99 strain, a Mar del Plata/595/99 strain, a Maryland/1/01 strain, a Memphis/1/01 strain, a Memphis/12/97-MA strain, a Michigan/22572/99 strain, a Mie/1/93 strain, a Milano/1/01 strain, a Minsk/318/90 strain, a Moscow/3/03 strain, a Nagoya/20/99 strain, a Nanchang/1/00 strain, a Nashville/107/93 strain, a Nashville/45/91 strain, a Nebraska/2/01 strain, a Netherland/801/90 strain, a Netherlands/429/98 strain, a New York/1/2002 strain, a NIB/48/90 strain, a Ningxia/45/83 strain, a Norway/1/84 strain, an Oman/16299/2001 strain, an Osaka/1059/97 strain, an Osaka/983/97-V2 strain, an Oslo/1329/2002 strain, an Oslo/1846/2002 strain, a Panama/45/90 strain, a Paris/329/90 strain, a Parma/23/02 strain, a Perth/211/2001 strain, a Peru/1364/2004 strain, a Philippines/5072/2001 strain, a Phuket/3073/2013 strain, a Pusan/270/99 strain, a Quebec/173/98 strain, a Quebec/465/98 strain, a Quebec/7/01 strain, a Roma/1/03 strain, a Saga/S172/99 strain, a Seoul/13/95 strain, a Seoul/37/91 strain, a Shandong/7/97 strain, a Shanghai/361/2002 strain, a Shiga/T30/98 strain, a Sichuan/379/99 strain, a Singapore/222/79 strain, a Spain/WV27/2002 strain, a Stockholm/10/90 strain, a Switzerland/5441/90 strain, a Taiwan/0409/00 strain, a Taiwan/0722/02 strain, a Taiwan/97271/2001 strain, a Tehran/80/02 strain, a Tokyo/6/98 strain, a Trieste/28/02 strain, a Ulan Ude/4/02 strain, a United Kingdom/34304/99 strain, a USSR/100/83 strain, a Victoria/103/89 strain, a Vienna/1/99 strain, a Wuhan/356/2000 strain, a WV194/2002 strain, a Xuanwu/23/82 strain, a Yamagata/1311/2003 strain, a Yamagata/K500/2001 strain, an Alaska/12/96 strain, a GA/86 strain, a NAGASAKI/1/87 strain, a Tokyo/942/96 strain, and a Rochester/02/2001 strain. The sequences of the above virus strains are known in the art and available from GenBank.

The hemagglutination inhibition (HAI) test is a classic laboratory procedure used to classify or type hemagglutinating viruses and further characterize the antigenic properties of influenza virus isolates. For HAI determination, serial dilutions of the virus are prepared in U-bottom or V-bottom 96-well microtiter plates. For example, the most concentrated sample in the first well may use a 1/5 concentration of the stock solution, and subsequent wells may be serially diluted two-fold (1/10, 1/20, 1/40, etc.). The last well serves as a negative control without any virus. Different rows of the plate typically have different viruses with the same dilution pattern. Following serial dilution, a standardized concentration of red blood cells (RBCs) is added to each well and mixed gently. The plate is incubated at room temperature. After the incubation period, the assay can be analyzed to distinguish between agglutinated and non-agglutinated wells. The relative concentration or titer of the virus sample is determined based on the well in which the last agglutination occurs before a precipitate is observed.

Serological methods such as the HAI assay can be used for epidemiological and immunological studies of viruses and vaccines, to evaluate antibody responses after vaccination, to measure the efficacy of candidate vaccines, and to identify antigenically similar influenza viruses. Viruses with HAI titers differing by a factor of two or less in dilution can be regarded as antigenically similar. In some examples, the mRNA vaccine disclosed herein has an HAI titer increased by 2-, 3-, 4-, 5-, 6-, 7-, 8-, 9-, or 10-fold relative to a control (e.g., compared with a subject administered a traditional quadrivalent influenza vaccine). In some instances, an HA ELISA assay is performed to examine HA antibody titers (e.g., IgG antibody titers) generated by administration of a candidate vaccine.

As used herein, "N:P ratio" or "N/P ratio" refers to the molar ratio of ionizable nitrogen atoms in the ionizable lipid to phosphate groups in the RNA.

### Examples

The present invention will be described in further detail below in conjunction with specific embodiments. The examples provided are intended only to illustrate the present invention, rather than to limit the scope of the present invention. Various modifications or adjustments may be made by those skilled in the art in light of the teachings of the present invention, without departing from the spirit and scope of the present invention.

### Biosafety

The present invention used 4 pathogenic influenza viruses: A/Puerto Rico/8/34 (H1N1), A/Aichi/2/1968 (X31) (H3N2), B/Brisbane/60/2008 (B/Victoria), and B/Phuket/3073/2013 (B/Yamagata). The experimental procedures were conducted in a Biosafety Level 2 laboratory at the Institute of Medicinal Biotechnology, Chinese Academy of Medical Sciences. The strains A/Puerto Rico/8/34 (H1N1), B/Brisbane/60/2008 (B/Victoria), and B/Phuket/3073/2013 (B/Yamagata) were obtained from the Institute of Medical Biology, Chinese Academy of Medical Sciences. The strain A/Aichi/2/1968 (X31) (H3N2) was obtained from the Chinese National Influenza Center, National Institute for Viral Disease Control and Prevention, Chinese Center for Disease Control and Prevention. All experimental materials, operations, and waste disposal complied with the safety regulations of the laboratory.

All experimental operations and waste disposal in the present invention complied with the relevant provisions of the WHO *Laboratory Biosafety Manual (Fourth Edition)*, the Chinese standard Laboratories - General requirements for biosafety (GB 19489-2008), and the Regulation on the Biosafety Management of Pathogenic Microbe Labs.

### Experimental Materials, Reagents, Instruments, and Experimental Methods

Unless otherwise specified, the reagents, instruments, genes, and enzymes used in the present invention are commercially available. The relevant nucleic acid strands, genes, and enzymes can also be obtained by conventional molecular biology experimental techniques based on information from public databases. Unless otherwise stated, the nucleotide sequences described in the specification are written in the 5' end to 3' end direction, and the amino acid sequences are written in the amino terminal to carboxy terminal direction. In case of any inconsistency between the sequences in the specification and the sequence listing, the sequences described in the specification shall prevail.

### Example 1:

Double-stranded DNA plasmids 1 to 7 for the preparation of an mRNA were commissioned to Suzhou Genewiz Biotechnology Co., Ltd. for synthesis.

Plasmids 1 to 7 include the nucleotide sequences set forth in SEQ ID NOs: 9, 11, 13, 15, 17, 19, and 21, respectively, which encode the immunogenic polypeptide BW having the amino acid sequence set forth in SEQ ID NO: 10, the chimeric immunogenic polypeptide 51-BW having the amino acid sequence set forth in SEQ ID NO: 12, the hemagglutinin HA of H3N2 having the amino acid sequence set forth in SEQ ID NO: 14, the hemagglutinin HA of H7N9 having the amino acid sequence set forth in SEQ ID NO: 16, the hemagglutinin HA of B/Yamagata having the amino acid sequence set forth in SEQ ID NO: 18, the M2 protein having the amino acid sequence set forth in SEQ ID NO: 20, and the 51 chimeric polypeptide having the amino acid sequence set forth in SEQ ID NO: 22, respectively.

According to the manufacturer's instructions, plasmids 1 to 7 were digested with the restriction enzyme SapI (purchased from NEB) using the reaction system shown in Table 1, and reacted at 37°C for 3 hours to obtain the corresponding linearized plasmid templates.

**Table 1: SapI restriction enzyme digestion reaction system**

| Reagent | Volume added |
|---|---|
| 10× Cutsmart Buffer | 10 µL |
| SapI enzyme (10,000 U/mL) | 6 µL |
| Plasmid | 10 µg |
| ddH₂O | Make up to 100 µL |

According to the manufacturer's instructions, the linearized plasmid templates above were purified and recovered using the linearized plasmid recovery and purification kit DP205-02 (purchased from TIANGEN). Specifically, 5 volumes of PB Buffer were added to the linearization reaction system and mixed thoroughly. The filter column was activated with 200 µL PS buffer, centrifuged at 12,000×g for 1 min, and the filtrate was discarded. The mixed reaction solution was loaded onto the column, centrifuged at 12,000×g for 1 min, and the filtrate was discarded. Impurities not bound to the column were washed away using 700 µL washing buffer and centrifugation at 12,000×g for 1 min. The linearized plasmid templates were eluted into a clean 1.5 mL EP tube with 50 µL RNase-free ddH₂O (12,000×g, 2 min) to complete purification. The purified linearized plasmids were quantified using Nanodrop.

According to the manufacturer's instructions, capping and *in vitro* transcription were performed on the purified linearized plasmid templates 1 to 7 above using T7 polymerase and a CleanCap reagent kit, respectively. Specifically, the capping and *in vitro* transcription reaction system shown in Table 2 below was added to a 1.5 mL EP tube, placed in a 37°C incubator, and reacted under rotation for 3 hours (10 rpm).

**Table 2: mRNA capping and in vitro transcription reaction system:**

| Reagent | Volume added |
|---|---|
| 10× Buffer | 50 µL |
| ATP (100 mM) | 50 µL |
| N1-Me-pUTP (100 mM) | 50 µL |
| CTP (100 mM) | 50 µL |
| GTP (100 mM) | 50 µL |
| T7 | 25 µL |
| IPP | 25 µL |
| RINeo | 25 µL |
| CleanCap AG (100 mM) | 25 µL |
| Linearized template | 1 µg-2 µg |
| Nuclease-free H₂O | Make up to 500 µL |

After completion of the *in vitro* transcription reaction, 20 µL of DNase I was added to the tube, mixed thoroughly by pipetting, and reacted at 37°C for 15 min. Then, 50% volume of LiCl was added to the reaction system, and the mixture was allowed to stand overnight at - 20°C. The mixture was transferred to a centrifuge pre-cooled to 4°C, and centrifuged at 12,000×g for 15 min, and the supernatant was discarded. After washing with 700 µL of 70% ethanol and centrifugation again (12,000×g, 5 min), the white precipitate was redissolved in 1,000 µL of RNase-free water, mixed thoroughly, and quantified with Nanodrop to determine the amount of the recovered mRNA.

The prepared mRNA includes the following elements in sequence from the 5' end to the 3' end: (1) a 5' cap; (2) a 5' UTR region (as set forth in SEQ ID NO: 23); (3) an antigen coding region; (4) a 3' UTR region (as set forth in SEQ ID NO: 24); and (5) a PolyA tail (as set forth in SEQ ID NO: 25).

### Example 2:

To the mRNA stock solution purified in Example 1, acetic acid solution was added to a final concentration of 20 mmol/L acetic acid and 200 µg/ml mRNA, and mixed thoroughly by stirring to serve as the mRNA working solution. The mRNA working solution and lipid mixture solution (formulated according to Table 3) were mixed at a flow ratio of 2:1 to 4:1 through a T-mixer to prepare LNPs. Then, the LNPs were diluted 2-5-fold with a 2 mmol/L acetic acid solution, followed by exchange with the 2 mmol/L acetic acid solution for at least 3 rounds, and the feed solution was concentrated to the target concentration. A sucrose solution was added to adjust the osmotic pressure, and a Tris solution was used to adjust the pH to 7.0-8.0 to obtain mRNA-lipid nanoparticles (LNPs) including the mRNA prepared and purified in Example 1.

In the prepared mRNA-LNPs, the N/P ratio of the mRNA to ionizable lipid was 5.2.

**Table 3: Lipid mixture solution formulation**

| Lipid mixture component | mol% |
|---|---|
| Ionizable lipid | 50 |
| Cholesterol | 38.5 |
| Phospholipid | 10 |
| DMG-PEG2000 | 1.5 |

The mRNA-LNPs prepared as described above were mixed at the mass ratios shown in Table 4 to prepare the mRNA-LNP compositions shown in each group.

**Table 4:**

| Group | mRNA-LNP composition | Mass ratio (based on mRNA) |
|---|---|---|
| G1 | Blank LNP | / |
| G2 | PBS | / |
| G3 | 51-BW (chimeric group) | 5 µg |
| G4 | 51 + BW (mixed group) | 2.5 µg: 2.5 µg |
| G5 | 51-BW:H3N2:H7N9:BP:M2 | 5µg :5µg:2µg:2µg:2µg |
| G6 | 51-BW:H3N2:H7N9:BP:M2 | 5µg :5µg:5µg:5µg:5µg |

| | | |
|---|---|---|
| Note: 1. 51-BW represents an mRNA encoding the 51-BW chimeric polypeptide; 2. 51 represents an mRNA encoding the 51 chimeric polypeptide; BW represents an mRNA encoding hemagglutinin HA of the B/Victoria strain; | | |

The molecular weight of the mRNA encoding the 51-BW chimeric polypeptide is approximately: 1.08×10⁶ Da, the molecular weight of the mRNA encoding the 51 chimeric polypeptide is approximately 5.23×10⁵ Da, and the molecular weight of the mRNA encoding the BW polypeptide is approximately 5.40×10⁵ Da; the molecular weights were calculated using SNAPGENE software.
3. H3N2 represents an mRNA encoding hemagglutinin HA of the H3N2 strain;
4. H7N9 represents an mRNA encoding hemagglutinin HA of the H7N9 strain;
5. BP represents an mRNA encoding hemagglutinin HA of the B/Yamagata strain;
6. M2 represents an mRNA encoding the M2 polypeptide.

### Example 3:

The mRNA-LNP compositions prepared in Example 2 were used to immunize 6-8-week-old female BALB/c mice (SPF grade), respectively, to evaluate the immune protective response against influenza virus induced by each composition.

Specifically, the mice were randomly divided into groups with 5 mice per group, and the mRNA-LNP compositions were administered by intramuscular injection (doses shown in Table 4). A booster injection was given on day 14. On day 28, approximately 200 µL of blood was collected from the mice by orbital bleeding. The blood was incubated in a 37°C water bath for 60 min, followed by refrigeration at 4°C for 120 min. After centrifugation at 3,000 rpm for 10 min, serum was separated for subsequent neutralizing antibody assays and hemagglutination inhibition tests.

### Example 4:

Mouse sera of each group prepared according to Example 3 were mixed with receptor-destroying enzyme (RDE) at a volume ratio of 1:3, incubated in a 37°C water bath for 12 h, and inactivated in a 56°C water bath for 30 min to terminate the reaction. Hemagglutination tests for H5N1 and H7N9 were performed using commercially purchased avian influenza virus H5 or H7 subtype hemagglutination inhibition test antigens (Harbin Weike Biotechnology Co., Ltd.).

PBS was added to a V-bottom 96-well plate at 25 µL/well, and 25 µL of virus stock solution was added to the first well and mixed thoroughly with PBS by pipetting. Then 25 µL of the mixed solution was aspirated from this well, added to the second well, and mixed, followed by serial 2-fold dilution for 12 gradients. Subsequently, 25 µL of 1% guinea pig blood was added simultaneously to each well containing the serially diluted virus solution, mixed thoroughly, and allowed to stand at room temperature for 30-45 min. The number of virus particles corresponding to the lowest concentration well in which hemagglutination was observed was taken as one hemagglutination unit. The virus dose used for the hemagglutination inhibition test was four hemagglutination units.

PBS was added to a V-bottom 96-well plate at 25 µL/well, and 25 µL of RDE-treated serum was added to the first well and mixed thoroughly with PBS by pipetting. Eight serial dilutions were performed vertically according to the same procedure as in section 1.2. Four hemagglutination units of diluted virus were added to each well at 25 µL/well, mixed thoroughly 1:1 with the serially diluted serum, and allowed to stand at room temperature for 1 h. Subsequently, 1% guinea pig blood was added to the above serum-virus mixed solution at 50 µL/well, mixed thoroughly, and allowed to stand at room temperature for 45 min to observe hemagglutination.

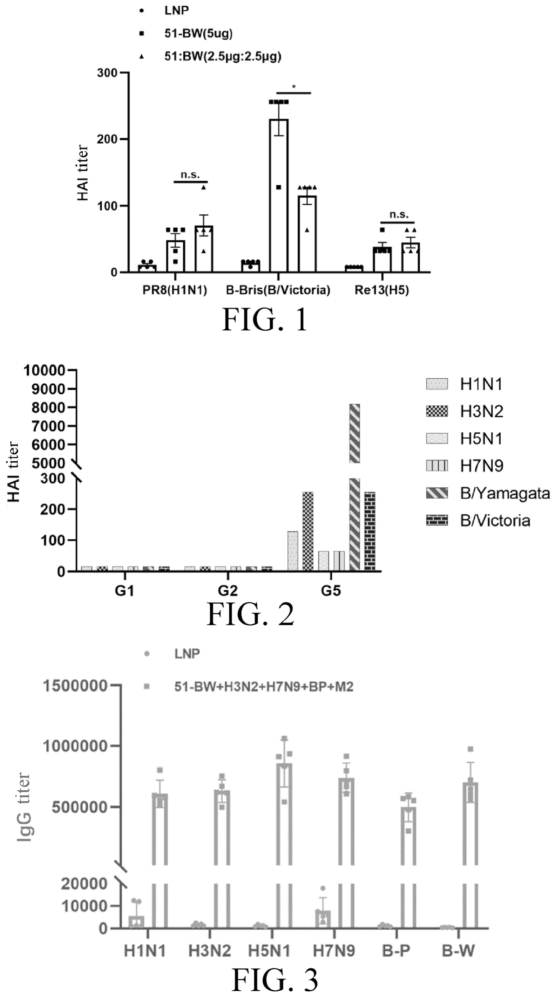

The final serum dilution factor corresponding to the well immediately preceding the first well in which observable hemagglutination occurred was taken as the hemagglutination titer. If hemagglutination was observed in the first well, the result was regarded as negative. The results are shown in FIG. 1 and FIG. 2.

FIG. 1 shows that, compared with the blank LNP control, the mRNA-LNP compositions of group G3 and group G4 both elicited significantly improved hemagglutination inhibition effects in the sera of immunized mice against H1, H5, and B-type hemagglutinin antigens. Further comparison of the results of group G3 and group G4 reveals that, compared with mice immunized with a mixture of 51 and BW (group G4), mice immunized with the exemplary chimeric immunogenic polypeptide 51-BW of this application alone (group G3) exhibited comparable hemagglutination inhibition effects against H1 and H5 hemagglutinin antigens, and group G3 showed a significantly superior hemagglutination inhibition effect against influenza B virus Victoria, with statistically significant differences. It indicates that further chimerization of the 51 chimeric protein with the BW protein can unexpectedly improve the immune effect *in vivo*.

FIG. 2 shows that sera of groups G1 and G2 as negative controls could not inhibit hemagglutination caused by any of the virus strains. In contrast, significant hemagglutination inhibition effects were observed in sera obtained from mice immunized with the mRNA-LNP composition of group G5, and the inhibition effects covered influenza A viruses H1N1, H3N2, H5N1, and H7N9 and influenza B viruses Yamagata and Victoria simultaneously. The above results further verify the potent and broad-spectrum immune protective activity of the chimeric antigen polypeptides and mRNA-LNP compositions disclosed herein. In addition, the results indicate that those skilled in the art can increase or decrease the types and ratios of influenza antigens in the composition according to actual conditions, and adjust the protective effect against individual virus strains without substantially changing the broad-spectrum immune protective activity.

### Example 5:

According to the manufacturer's instructions, an ELISA kit (Beijing Sino Biological Inc.) was used to determine antibody titers in mouse sera after immunization of mice with the mRNA-LNP compositions. Each antigen shown in Table 5 (all purchased from Sino Biological) was diluted to 2 µg/mL with 1× coating buffer (pH 9.0) and added to a 96-well microplate at 100 µL/well. The plate was sealed with a sealing film and incubated overnight at 4°C. Then, the supernatant was discarded, and the plate was washed twice with a washing buffer at 200 µL/well. A blocking buffer was added, and the plate was allowed to stand for 2 h, followed by air-drying at room temperature to obtain a coated 96-well plate. The coated plate may be sealed with a sealing film and stored with a desiccant in a refrigerator until use.

**Table 5**

| Antigen reagent | Source strain |
|---|---|
| H1N1 protein | A/Wisconsin/588/2019 |
| H3N2 protein | A/Cambodia/e0826360/2020 |
| H5N1 protein | A/Vietnam/1194/2004 |
| H7N9 protein | A/Zhejiang/DTID-ZJU10/2013 |
| BW protein | B/Washington/02/2019 |
| BP protein | B/PHUKET/3073/2013 |

Prior to use, the coated microplate was washed once with a washing buffer at 200 µL/well. Sera from mice in group G6 prepared according to Example 3 were diluted with a dilution buffer and then added to the microplate at 100 µL per well. The plate was covered with a sealing film and incubated at 37°C for 2 h. Then, the serum was discarded, and the plate was washed 4 times with a washing buffer at 200 µL/well, followed by addition of 100 µL/well of mouse secondary antibody at a ratio of 1:2,500. The plate was sealed with a sealing film and incubated at 37°C for 1 h. The secondary antibody was then discarded, and the plate was washed 4 times with a washing buffer at 200 µL/well. A chromogenic solution was added at 100 µL/well, the plate was allowed to stand in the dark at room temperature for 20 min, and then 50 µL/well of stop solution (diluted one-fold) was added to terminate color development. The measured value at a wavelength of 450 nm was read. The results are shown in FIG. 3.

FIG. 3 shows that immunization of mice using the mRNA-LNP composition of group G6 not only induced significantly elevated total anti-influenza virus IgG in mice, but also resulted in comparable proportions of IgG against antigens of six different virus strains (covering influenza A viruses H1N1, H3N2, H5N1, and H7N9 and common influenza B viruses Yamagata and Victoria). This indicates that the mRNA compositions disclosed herein can efficiently express all antigens including the trivalent chimeric antigen polypeptide *in vivo*, thereby successfully inducing an immune response against all parental antigens constituting the chimeric polypeptide. This can greatly reduce the dosage of the mRNA administered. It also indicates that after the chimeric antigen polypeptide herein is formulated into a mixture or composition with other influenza virus antigens, the expression of the chimeric antigen polypeptide and the immunogenicity of each constituent antigen unit contained therein are not adversely affected by other influenza virus antigens, nor do they interfere with the expression and immunogenicity of other influenza virus antigens, showing potential for being formulated into multivalent influenza vaccines (especially multivalent vaccines against both influenza A and B viruses), thereby achieving a broad-spectrum immune effect against influenza viruses.

The sequences used in the above examples of this application are shown below. It should be understood that the following sequences are merely exemplary sequences of the implementations of this application, rather than any limitation to the solutions of this application. The nucleic acid sequences in the sequence listing below may represent DNA sequences or RNA sequences. When they represent RNA sequences, "T" stands for uridine.
SEQ ID NO. 1 (nucleotide sequence of the folding region) 
ATGAAGGCCATCCTGGTGGTGATGCTGTACACCTTCACCACCGCCAACGCCGACA
CCCTGTGCATCGGCTACCACGCCAACAACAGCACCGACACCGTGGACACCGTGCT
GGAGAAGAACGTGACCGTGACCCACAGCGTGAACCTGCTGGAGGACAAGCACA
ACGGCAAGCTG
SEQ ID NO. 2 (amino acid sequence of the folding region) 
MKAILVVMLYTFTTANADTLCIGYHANNSTDTVDTVLEKNVTVTHSVNLLEDKHNG
KL
SEQ ID NO. 3 (nucleotide sequence of the HA head of H5N1) 
TGTGACCTGGACGGCGTGAAACCTCTGATCCTGAGAGACTGCAGCGTGGCCGGCT
GGCTGCTGGGCAACCCCATGTGCGACGAGTTCATCAACGTGCCCGAGTGGAGCTA
CATCGTGGAGAAGGCCAACCCCGTGAACGACCTGTGCTACCCCGGCGACTTCAAC
GACTACGAGGAGCTGAAGCACCTGCTGAGCAGAATCAACCACTTCGAGAAGATT
CAGATCATCCCCAAGAGCAGCTGGAGCAGCCACGAGGCTAGCCTGGGCGTGAGC
AGCGCCTGCCCCTACCAAGGCAAGAGCAGCTTCTTCAGAAACGTGGTGTGGCTG
ATCAACAAGAACAGCACCTACCCCACCATCAAGAGAAGCTACAACAACACCAAC
CAAGAGGACCTGCTGGTGCTGTGGGGCATCCACCACCCCAACGACGCCGCCGAG
CAGACCAAGCTGTATCAGAATCCTACCACCTACATCAGCGTGGGCACAAGCACCC
TGAATCAGAGACTGGTGCCTAGAATCGCCACAAGATCCAAGGTGAACGGGCAGA
GCGGCAGAATGGAGTTCTTCTGGACCATCCTGAAGCCTAACGACGCCATCAACTT
CGAGAGCAACGGCAACTTCATCGCCCCCGAGTACGCCTACAAGATCGTGAAGAA
GGGCGACAGCACCATCATGAAAAGCGAGCTGGAGTACGGCAACTGC
SEQ ID NO. 4 (amino acid sequence of the HA head of H5N1) CDLDGVKPLILRDCSVAGWLLGNPMCDEFINVPEWSYIVEKANPVNDLCYPGDFND 
YEELKHLLSRINHFEKIQIIPKSSWSSHEASLGVSSACPYQGKSSFFRNVVWLINKNST
YPTIKRSYNNTNQEDLLVLWGIHHPNDAAEQTKLYQNPTTYISVGTSTLNQRLVPRIA
TRSKVNGQSGRMEFFWTILKPNDAINFESNGNFIAPEYAYKIVKKGDSTIMKSELEYG
NC
SEQ ID NO. 5 (nucleotide sequence of the HA stem of H1N1) 
AACACCACCTGTCAGACCCCCGAGGGCGCCATCAACACAAGCCTGCCCTTTCAGA
ACGTGCACCCCATCACCATCGGCAAGTGCCCTAAGTACGTCAAGAGCACCAAGCT
GAGACTGGCCACCGGCCTGAGAAACGTGCCTAGCATTCAGAGCAGAGGCCTGTT
CGGCGCCATCGCCGGCTTCATCGAAGGCGGCTGGACCGGCATGGTGGACGGCTGG
TACGGCTACCACCATCAGAACGAGCAAGGCAGCGGCTACGCCGCCGATCTCAAGA
GCACACAGAACGCCATCGACAAGATCACCAACAAGGTGAACAGCGTGATCGAGA
AGATGAACACACAGTTCACCGCCGTGGGCAAGGAGTTCAACCACCTGGAGAAGA
GAATCGAGAACCTGAATAAAAAGGTGGACGACGGCTTCCTCGACATCTGGACCTA
CAACGCCGAGCTGCTGGTGCTGCTGGAGAACGAGAGAACCCTGGACTACCATGA
CAGCAACGTGAAGAACCTCTACGAGAAGGTGAGAAATCAGCTGAAGAACAACGC
CAAGGAGATCGGCAACGGGTGCTTCGAGTTCTACCACAAGTGCGACAACACCTG
CATGGAGAGCGTGAAGAACGGCACCTACGACTACCCCAAGTACAGCGAGGAGGC
CAAGCTGAACAGAGAGAAGATCGACGGCGTGAAGCTGGACAGCACAAGAATCTA
TCAGATCCTGGCCATCTACAGCACCGTGGCTAGCAGCCTGGTGCTCGTGGTGAGC
CTGGGCGCCATCAGCTTCTGGATGTGCAGCAATGGCAGCCTGCAGTGTCGGATCT
GCATC
SEQ ID NO. 6 (amino acid sequence of the HA stem of H1N1) 
NTTCQTPEGAINTSLPFQNVHPITIGKCPKYVKSTKLRLATGLRNVPSIQSRGLFGAIA
GFIEGGWTGMVDGWYGYHHQNEQGSGYAADLKSTQNAIDKITNKVNSVIEKMNTQ
FTAVGKEFNHLEKRIENLNKKVDDGFLDIWTYNAELLVLLENERTLDYHDSNVKNLY
EKVRNQLKNNAKEIGNGCFEFYHKCDNTCMESVKNGTYDYPKYSEEAKLNREKIDG
VKLDSTRIYQILAIYSTVASSLVLVVSLGAISFWMCSNGSLQCRICI
SEQ ID NO. 7 (nucleotide sequence of the flexible linker region)
   GGCGGGGGCGGCTCCGGCGGCGGGGGCAGCGGCGGGGGCGGCAGC
SEQ ID NO. 8 (amino acid sequence of the flexible linker region)
   GGGGSGGGGSGGGGS
SEQ ID NO. 9 (nucleotide sequence of the HA coding region of BW) 
ATGAAGGCTATCATCGTGCTGCTGATGGTGGTGACAAGCAACGCCGACAGAATCT
GCACCGGCATCACAAGCAGCAACAGCCCCCACGTGGTGAAGACCGCCACCCAAG
 
GCGAGGTGAACGTGACCGGCGTGATCCCCCTGACCACAACCCCCACCAAGAGCC
ACTTCGCCAACCTGAAGGGCACCGAGACAAGAGGCAAGCTGTGCCCCAAGTGCC
TGAACTGCACCGACCTGGACGTGGCCCTGGGCAGACCCAAGTGCACCGGCAAGA
TCCCTAGCGCTAGAGTGAGCATCCTGCACGAGGTGAGACCCGTGACAAGCGGCTG
CTTCCCCATCATGCACGACAGAACCAAGATCAGACAGCTGCCCAACCTGCTGAGA
GGCTACGAGCACGTGAGACTGAGCACCCACAACGTGATCAACGCCGAGGACGCC
CCCGGCAGACCCTACGAGATCGGCACAAGCGGCAGCTGCCCCAACATCACCAAC
GGCAACGGCTTCTTCGCCACCATGGCCTGGGCCGTGCCCAAGAACAAAACCGCC
ACCAACCCCCTGACCATCGAGGTGCCCTACATCTGCACCGAGGGCGAGGATCAGA
TCACCGTGTGGGGCTTCCACAGCGACAGCGAGACACAGATGGCCAAGCTGTACG
GCGACAGCAAGCCTCAGAAGTTCACAAGCAGCGCCAACGGCGTGACCACCCACT
ACGTGAGCCAAATCGGCGGCTTCCCCAATCAGACCGAGGACGGCGGCCTGCCTC
AGTCCGGGAGAATCGTGGTGGACTACATGGTGCAAAAGAGCGGCAAGACCGGCA
CCATCACCTATCAGAGAGGCATCCTGCTGCCTCAGAAGGTGTGGTGCGCTAGCGG
CAGAAGCAAGGTGATTAAGGGCAGCCTGCCCCTGATCGGCGAGGCCGACTGCCT
GCACGAGAAGTACGGCGGCCTGAACAAGAGCAAGCCCTACTACACCGGCGAGCA
CGCCAAGGCCATCGGCAACTGCCCCATCTGGGTGAAGACCCCCCTGAAGCTGGCC
AACGGCACCAAGTACAGACCCCCCGCCAAGCTGCTGAAGGAGAGAGGCTTCTTC
GGCGCCATTGCCGGCTTCCTGGAGGGCGGCTGGGAGGGCATGATCGCTGGCTGGC
ACGGCTATACAAGCCACGGCGCTCACGGCGTGGCTGTGGCTGCCGACCTCAAGAG
CACCCAAGAGGCTATCAACAAGATTACCAAAAACCTGAACTCCCTGAGCGAGCTC
GAGGTCAAGAACCTGCAGAGACTGAGCGGCGCCATGGACGAGCTGCACAACGAG
ATCCTGGAGCTGGACGAGAAGGTGGATGACCTGAGAGCCGACACCATCAGCAGC
CAAATCGAGCTGGCCGTGCTGCTGAGCAACGAGGGCATCATCAACAGCGAGGAC
GAGCACCTGCTGGCCCTGGAGAGAAAGCTGAAGAAGATGCTGGGCCCTAGCGCC
GTGGAGATTGGCAACGGCTGTTTCGAGACCAAGCACAAGTGCAATCAGACCTGCC
TGGACAGAATCGCCGCCGGCACCTTCGACGCCGGCGAGTTCAGCCTGCCCACCTT
CGACAGCCTGAACATCACCGCCGCTAGCCTGAACGACGACGGCCTGGACAACCA
CACCATCCTGCTGTACTACAGCACAGCCGCTAGCTCCCTGGCCGTGACCCTGATGA
TCGCCATCTTCGTGGTGTACATGGTGAGCAGAGACAACGTGAGCTGCAGCATCTG
CCTGTGA
SEQ ID NO. 10 (amino acid sequence of the HA coding region of BW) 
MKAIIVLLMVVTSNADRICTGITSSNSPHVVKTATQGEVNVTGVIPLTTTPTKSHFANL
KGTETRGKLCPKCLNCTDLDVALGRPKCTGKIPSARVSILHEVRPVTSGCFPIMHDRT
 
KIRQLPNLLRGYEHVRLSTHNVINAEDAPGRPYEIGTSGSCPNITNGNGFFATMAWAV
PKNKTATNPLTIEVPYICTEGEDQITVWGFHSDSETQMAKLYGDSKPQKFTSSANGVT
THYVSQIGGFPNQTEDGGLPQSGRIVVDYMVQKSGKTGTITYQRGILLPQKVWCASG
RSKVIKGSLPLIGEADCLHEKYGGLNKSKPYYTGEHAKAIGNCPIWVKTPLKLANGT
KYRPPAKLLKERGFFGAIAGFLEGGWEGMIAGWHGYTSHGAHGVAVAADLKSTQEA
INKITKNLNSLSELEVKNLQRLSGAMDELHNEILELDEKVDDLRADTISSQIELAVLLS
NEGIINSEDEHLLALERKLKKMLGPSAVEIGNGCFETKHKCNQTCLDRIAAGTFDAGE
FSLPTFDSLNITAASLNDDGLDNHTILLYYSTAASSLAVTLMIAIFVVYMVSRDNVSCS
ICL
SEQ ID NO. 11 (nucleotide sequence of 51-BW) 
ATGAAGGCCATCCTGGTGGTGATGCTGTACACCTTCACCACCGCCAACGCCGACA
CCCTGTGCATCGGCTACCACGCCAACAACAGCACCGACACCGTGGACACCGTGCT
GGAGAAGAACGTGACCGTGACCCACAGCGTGAACCTGCTGGAGGACAAGCACA
ACGGCAAGCTG
TGTGACCTGGACGGCGTGAAACCTCTGATCCTGAGAGACTGCAGCGTGGCCGGCT
GGCTGCTGGGCAACCCCATGTGCGACGAGTTCATCAACGTGCCCGAGTGGAGCTA
CATCGTGGAGAAGGCCAACCCCGTGAACGACCTGTGCTACCCCGGCGACTTCAAC
GACTACGAGGAGCTGAAGCACCTGCTGAGCAGAATCAACCACTTCGAGAAGATT
CAGATCATCCCCAAGAGCAGCTGGAGCAGCCACGAGGCTAGCCTGGGCGTGAGC
AGCGCCTGCCCCTACCAAGGCAAGAGCAGCTTCTTCAGAAACGTGGTGTGGCTG
ATCAACAAGAACAGCACCTACCCCACCATCAAGAGAAGCTACAACAACACCAAC
CAAGAGGACCTGCTGGTGCTGTGGGGCATCCACCACCCCAACGACGCCGCCGAG
CAGACCAAGCTGTATCAGAATCCTACCACCTACATCAGCGTGGGCACAAGCACCC
TGAATCAGAGACTGGTGCCTAGAATCGCCACAAGATCCAAGGTGAACGGGCAGA
GCGGCAGAATGGAGTTCTTCTGGACCATCCTGAAGCCTAACGACGCCATCAACTT
CGAGAGCAACGGCAACTTCATCGCCCCCGAGTACGCCTACAAGATCGTGAAGAA
GGGCGACAGCACCATCATGAAAAGCGAGCTGGAGTACGGCAACTGC
AACACCACCTGTCAGACCCCCGAGGGCGCCATCAACACAAGCCTGCCCTTTCAGA
ACGTGCACCCCATCACCATCGGCAAGTGCCCTAAGTACGTCAAGAGCACCAAGCT
GAGACTGGCCACCGGCCTGAGAAACGTGCCTAGCATTCAGAGCAGAGGCCTGTT
CGGCGCCATCGCCGGCTTCATCGAAGGCGGCTGGACCGGCATGGTGGACGGCTGG
TACGGCTACCACCATCAGAACGAGCAAGGCAGCGGCTACGCCGCCGATCTCAAGA
GCACACAGAACGCCATCGACAAGATCACCAACAAGGTGAACAGCGTGATCGAGA
AGATGAACACACAGTTCACCGCCGTGGGCAAGGAGTTCAACCACCTGGAGAAGA
 
GAATCGAGAACCTGAATAAAAAGGTGGACGACGGCTTCCTCGACATCTGGACCTA
CAACGCCGAGCTGCTGGTGCTGCTGGAGAACGAGAGAACCCTGGACTACCATGA
CAGCAACGTGAAGAACCTCTACGAGAAGGTGAGAAATCAGCTGAAGAACAACGC
CAAGGAGATCGGCAACGGGTGCTTCGAGTTCTACCACAAGTGCGACAACACCTG
CATGGAGAGCGTGAAGAACGGCACCTACGACTACCCCAAGTACAGCGAGGAGGC
CAAGCTGAACAGAGAGAAGATCGACGGCGTGAAGCTGGACAGCACAAGAATCTA
TCAGATCCTGGCCATCTACAGCACCGTGGCTAGCAGCCTGGTGCTCGTGGTGAGC
CTGGGCGCCATCAGCTTCTGGATGTGCAGCAATGGCAGCCTGCAGTGTCGGATCT
GCATCGGCGGGGGCGGCTCCGGCGGGGGCAGCGGCGGGGGCGGCAGCATG
AAGGCTATCATCGTGCTGCTGATGGTGGTGACAAGCAACGCCGACAGAATCTGCA
CCGGCATCACAAGCAGCAACAGCCCCCACGTGGTGAAGACCGCCACCCAAGGCG
AGGTGAACGTGACCGGCGTGATCCCCCTGACCACAACCCCCACCAAGAGCCACT
TCGCCAACCTGAAGGGCACCGAGACAAGAGGCAAGCTGTGCCCCAAGTGCCTGA
ACTGCACCGACCTGGACGTGGCCCTGGGCAGACCCAAGTGCACCGGCAAGATCC
CTAGCGCTAGAGTGAGCATCCTGCACGAGGTGAGACCCGTGACAAGCGGCTGCTT
CCCCATCATGCACGACAGAACCAAGATCAGACAGCTGCCCAACCTGCTGAGAGG
CTACGAGCACGTGAGACTGAGCACCCACAACGTGATCAACGCCGAGGACGCCCC
CGGCAGACCCTACGAGATCGGCACAAGCGGCAGCTGCCCCAACATCACCAACGG
CAACGGCTTCTTCGCCACCATGGCCTGGGCCGTGCCCAAGAACAAAACCGCCACC
AACCCCCTGACCATCGAGGTGCCCTACATCTGCACCGAGGGCGAGGATCAGATCA
CCGTGTGGGGCTTCCACAGCGACAGCGAGACACAGATGGCCAAGCTGTACGGCG
ACAGCAAGCCTCAGAAGTTCACAAGCAGCGCCAACGGCGTGACCACCCACTACG
TGAGCCAAATCGGCGGCTTCCCCAATCAGACCGAGGACGGCGGCCTGCCTCAGTC
CGGGAGAATCGTGGTGGACTACATGGTGCAAAAGAGCGGCAAGACCGGCACCAT
CACCTATCAGAGAGGCATCCTGCTGCCTCAGAAGGTGTGGTGCGCTAGCGGCAGA
AGCAAGGTGATTAAGGGCAGCCTGCCCCTGATCGGCGAGGCCGACTGCCTGCACG
AGAAGTACGGCGGCCTGAACAAGAGCAAGCCCTACTACACCGGCGAGCACGCCA
AGGCCATCGGCAACTGCCCCATCTGGGTGAAGACCCCCCTGAAGCTGGCCAACG
GCACCAAGTACAGACCCCCCGCCAAGCTGCTGAAGGAGAGAGGCTTCTTCGGCG
CCATTGCCGGCTTCCTGGAGGGCGGCTGGGAGGGCATGATCGCTGGCTGGCACGG
CTATACAAGCCACGGCGCTCACGGCGTGGCTGTGGCTGCCGACCTCAAGAGCACC
CAAGAGGCTATCAACAAGATTACCAAAAACCTGAACTCCCTGAGCGAGCTCGAG
GTCAAGAACCTGCAGAGACTGAGCGGCGCCATGGACGAGCTGCACAACGAGATC
CTGGAGCTGGACGAGAAGGTGGATGACCTGAGAGCCGACACCATCAGCAGCCAA
 
ATCGAGCTGGCCGTGCTGCTGAGCAACGAGGGCATCATCAACAGCGAGGACGAG
CACCTGCTGGCCCTGGAGAGAAAGCTGAAGAAGATGCTGGGCCCTAGCGCCGTG
GAGATTGGCAACGGCTGTTTCGAGACCAAGCACAAGTGCAATCAGACCTGCCTGG
ACAGAATCGCCGCCGGCACCTTCGACGCCGGCGAGTTCAGCCTGCCCACCTTCGA
CAGCCTGAACATCACCGCCGCTAGCCTGAACGACGACGGCCTGGACAACCACAC
CATCCTGCTGTACTACAGCACAGCCGCTAGCTCCCTGGCCGTGACCCTGATGATCG
CCATCTTCGTGGTGTACATGGTGAGCAGAGACAACGTGAGCTGCAGCATCTGCCT
GTGA
SEQ ID NO. 12 (amino acid sequence of 51-BW) 
MKAILVVMLYTFTTANADTLCIGYHANNSTDTVDTVLEKNVTVTHSVNLLEDKHNG
KLCDLDGVKPLILRDCSVAGWLLGNPMCDEFINVPEWSYIVEKANPVNDLCYPGDFN
DYEELKHLLSRINHFEKIQIIPKSSWSSHEASLGVSSACPYQGKSSFFRNVVWLINKNS
TYPTIKRSYNNTNQEDLLVLWGIHHPNDAAEQTKLYQNPTTYISVGTSTLNQRLVPRI
ATRSKVNGQSGRMEFFWTILKPNDAINFESNGNFIAPEYAYKIVKKGDSTIMKSELEY
GNCNTTCQTPEGAINTSLPFQNVHPITIGKCPKYVKSTKLRLATGLRNVPSIQSRGLFG
AIAGFIEGGWTGMVDGWYGYHHQNEQGSGYAADLKSTQNAIDKITNKVNSVIEKM
NTQFTAVGKEFNHLEKRIENLNKKVDDGFLDIWTYNAELLVLLENERTLDYHDSNVK
NLYEKVRNQLKNNAKEIGNGCFEFYHKCDNTCMESVKNGTYDYPKYSEEAKLNRE
KIDGVKLDSTRIYQILAIYSTVASSLVLVVSLGAISFWMCSNGSLQCRICIGGGGSGGG
GSGGGGSMKAIIVLLMVVTSNADRICTGITSSNSPHVVKTATQGEVNVTGVIPLTTTPT
KSHFANLKGTETRGKLCPKCLNCTDLDVALGRPKCTGKIPSARVSILHEVRPVTSGCF
PIMHDRTKIRQLPNLLRGYEHVRLSTHNVINAEDAPGRPYEIGTSGSCPNITNGNGFFA
TMAWAVPKNKTATNPLTIEVPYICTEGEDQITVWGFHSDSETQMAKLYGDSKPQKFTS
SANGVTTHYVSQIGGFPNQTEDGGLPQSGRIVVDYMVQKSGKTGTITYQRGILLPQK
VWCASGRSKVIKGSLPLIGEADCLHEKYGGLNKSKPYYTGEHAKAIGNCPIWVKTPL
KLANGTKYRPPAKLLKERGFFGAIAGFLEGGWEGMIAGWHGYTSHGAHGVAVAADL
KSTQEAINKITKNLNSLSELEVKNLQRLSGAMDELHNEILELDEKVDDLRADTISSQIE
LAVLLSNEGIINSEDEHLLALERKLKKMLGPSAVEIGNGCFETKHKCNQTCLDRIAAG
TFDAGEFSLPTFDSLNITAASLNDDGLDNHTILLYYSTAASSLAVTLMIAIFVVYMVSR
DNVSCSICL
SEQ ID NO. 13 (nucleotide sequence of the HA coding region of H3N2) 
ATGAAAACGATTATTGCGCTGAGCAACATTCTGTGCCTGGTGTTTGCGCAAAAGAT
TCCGGGCAACGATAACAGCACCGCGACCCTGTGCCTGGGCCATCATGCGGTGCCG
AACGGCACCGTGGTGAAAACCATTACCAACGATCGCATTGAAGTGACCAACGCGA
 
CCGAACTGGTGCAGAACAGCAGCATTGGCGAAATTTGCAACAGCCCGCATCAGAT
TCTGGATGGCGGCAACTGCACCCTGATTGATGCGCTGCTGGGCGATCCGCAGTGC
GATGGCTTTCAGAACAAAGAATGGGATCTGTTTGTGGAACGCAGCCGCGCGAACA
GCAGCTGCTATCCGTATGATGTGCCGGATTATGCGAGCCTGCGCAGCCTGGTGGCG
AGCAGCGGCACCCTGGAATTTAAAAACGAAAGCTTTAACTGGACCGGCGTGAAA
CAGAACGGCACGAGCAGCGCGTGCATTCGCGGCAGCAGTAGCAGCTTTTTTAGCC
GCCTGAACTGGCTGACGAGCCTGAACAACATTTATCCGGCGCAGAACGTGACCAT
GCCGAACAAAGAACAGTTTGATAAACTGTATATTTGGGGCGTGCATCATCCGGATA
CCGATAAAAATCAGTTTAGCCTGTTTGCGCAGAGCAGCGGCCGCATTACCGTGAG
CACCAAACGCAGTCAGCAAGCGGTGATTCCGAACATTGGCAGCCGCCCGCGCATT
CGCGATATTCCGAGCCGCATTAGCATTTATTGGACCATTGTGAAACCGGGCGATATT
CTGCTGATTAACAGCACCGGCAACCTGATTGCGCCGCGCGGCTATTTTAAAATTCG
CAGCGGCAAAAGCAGCATTATGCGCAGCGATGCGCCGATTGGCAAATGCAAAAGC
GAATGCATTACCCCGAACGGCAGCATTCCGAACGATAAACCGTTTCAGAACGTGA
ACCGCATTACCTATGGCGCGTGCCCGCGCTATGTGAAACAGAGCACCCTGAAACT
GGCGACCGGCATGCGCAACGTGCCGGAAAAACAGACCCGCGGCATTTTTGGCGC
GATTGCGGGCTTTATTGAAAACGGCTGGGAAGGCATGGTGGATGGCTGGTATGGC
TTTCGCCATCAGAACAGCGAAGGCCGCGGCCAAGCGGCGGATCTGAAAAGCACC
CAAGCGGCGATTGATCAGATTAGCGGCAAACTGAACCGCCTGATTGGCAAAACCA
ACGAAAAATTTCATCAGATTGAAAAAGAATTTAGCGAAGTGGAAGGCCGCGTGCA
AGATCTGGAAAAATATGTGGAAGATACCAAAATTGATCTGTGGAGCTATAACGCGG
AACTGCTGGTGGCGCTGGAAAATCAGCATACCATTGATCTGACCGATAGCGAAAT
GAACAAACTGTTTGAAAAAACCAAAAAACAGCTGCGCGAAAACGCGGAAGATAT
GGGCAACGGCTGCTTTAAAATTTATCATAAATGCGATAACGCGTGCATTGGCAGCA
TTCGCAACGAAACCTATGATCATAACGTGTATCGCGATGAAGCGCTGAACAACCGC
TTTCAGATTAAAGGCGTGGAACTGAAAAGCGGCTATAAAGATTGGATTCTGTGGAT
TAGCTTTGCGATGAGCTGCTTTCTGCTGTGCATTGCGCTGTTAGGCTTTATTATGTG
GGCGTGTCAGAAAGGCAACATTCGCTGCAACATTTGCATTTAA
SEQ ID NO. 14 (amino acid sequence of the HA coding region of H3N2) 
MKTIIALSNILCLVFAQKIPGNDNSTATLCLGHHAVPNGTVVKTITNDRIEVTNATELV
QNSSIGEICNSPHQILDGGNCTLIDALLGDPQCDGFQNKEWDLFVERSRANSSCYPYD
VPDYASLRSLVASSGTLEFKNESFNWTGVKQNGTSSACIRGSSSSFFSRLNWLTSLNNI
YPAQNVTMPNKEQFDKLYIWGVHHPDTDKNQFSLFAQSSGRITVSTKRSQQAVIPNIG
SRPRIRDIPSRISIYWTIVKPGDILLINSTGNLIAPRGYFKIRSGKSSIMRSDAPIGKCKSE
 
CITPNGSIPNDKPFQNVNRITYGACPRYVKQSTLKLATGMRNVPEKQTRGIFGAIAGFI
ENGWEGMVDGWYGFRHQNSEGRGQAADLKSTQAAIDQISGKLNRLIGKTNEKFHQI
EKEFSEVEGRVQDLEKYVEDTKIDLWSYNAELLVALENQHTIDLTDSEMNKLFEKTK
KQLRENAEDMGNGCFKIYHKCDNACIGSIRNETYDHNVYRDEALNNRFQIKGVELK
SGYKDWILWISFAMSCFLLCIALLGFIMWACQKGNIRCNICI*
SEQ ID NO. 15 (nucleotide sequence of the HA coding region of H7N9) 
ATGAACACACAGATCCTGGTGTTCGCCCTGGTGGTCGTGATCCCCACAAGCGCCG
ACAAGATCTGCCTGGGCCACCACGCCGTGAGCAACGGCACAAGAGTGAACACCC
TGACCGAGAGAGGCGTGGAGGTGGTGAACGCCACCGAGACCGTGGAGAGAACC
AACGTGCCTAGAATCTGCAGCAAGGGCAAGAGAACCATCGATCTGGGCCAATGCG
GCCTGCTGGGCACCATCACCGGCCCCCCTCAGTGCGATCAGTTCCTGGAGTTCAG
CGCCGACCTGATCATCGAGAGAAGAGAGGGCAGCGACGTGTGCTACCCCGGCAA
GTTCGTGAACGAGGAGGCCCTGAGACAGATCCTGAGAGAGAGCGGCGGCATCGA
CAAGGAGACCATGGGCTTCACCTACAGCGGCATCAGAACCAACGGCGCCACCAA
CGCCTGCAGAAGAAGCGGCAGCAGCTTCTACGCCGAGATGAAGTGGCTGCTGAG
CAACACCGACAACGCCGCCTTCCCTCAGATGACCAAGAGCTACAAGAACACAAG
AAGAGACCCCGCCCTGATCGTGTGGGGCGTGCACCACAGCGGCAGCACCACCGA
GCAGACCAAGCTGTACGGCAGCGGCAGCAAGCTGATCACCGTGGGCAGCAGCAA
CTATCAGCAGAGCTTCGTGCCTAGCCCCGGCGCTAGACCCCAAGTGAACGGGCAG
AGCGGCAGAATCGACTTCCACTGGCTGATCCTGGACCCCAACGACACCGTGACCT
TCAGCTTCAACGGCGCCTTCATCGCCCCCGACAGAGCTAGCTTCCTGAGAGGCAA
GAGCATGGGCATTCAGAGCGGCGTGCAAGTGGACGCCAACTGCGAGGGCGACTG
CTACCACAGCGGCGGCACCATCATCAGCAACCTGCCCTTTCAGAACATCAACAGC
AGAGCCGTGGGCAAGTGCCCTAGATACGTGAAGCAAGAGAGCCTGATGCTGGCC
ACCGGCATGAAGAACGTGCCCGAGCTGCCCAAGGGCAGAGGCCTGTTCGGCGCC
ATCGCCGGCTTCATCGAGAACGGCTGGGAGGGCCTGATCGACGGCTGGTACGGCT
TCAGACATCAGAACGCCCAAGGCGAGGGCACCGCCGCCGACTACAAGAGCACAC
AGAGCGCCATCGATCAGATCACCGGCAAGCTGAACAGACTGATCGAGAAGACCA
ATCAGCAGTTCGAGCTGATCGACAACGAGTTCACCGAGGTGGAGAAGCAGATCG
GCAACGTGATCAACTGGACAAGAGACAGCATGACCGAGGTGTGGAGCTACAACG
CCGAGCTGCTGGTGGCCATGGAGAATCAGCACACCATCGACCTCACAGACAGCG
AGATGAACAAGCTGTACGAGAGAGTGAGAAGACAGCTGAGAGAGAACGCCGAG
GAGGACGGCACCGGCTGCTTCGAGATCTTCCACAAGTGCGACGACAACTGCATG
GCTAGCATCAGAAACAACACCTACGACCACAGCAAGTACAGAGAGGAGGCCATG
 
CAGAACAGAATTCAGATCGACCCCGTGAAGCTGAGCAGCGGCTACAAGGACGTG
ATCCTGTGGTTCAGCTTCGGCGCTAGCTGCTTCATCCTGCTGGCCATCGCCATGGG
CCTGGTGTTCATCTGCGTGAAGAACGGCAACATGAGATGCACCATCTGCATCTGA
SEQ ID NO. 16 (amino acid sequence of the HA coding region of H7N9) 
MNTQILVFALVVVIPTSADKICLGHHAVSNGTRVNTLTERGVEVVNATETVERTNVPRI
CSKGKRTIDLGQCGLLGTITGPPQCDQFLEFSADLIIERREGSDVCYPGKFVNEEALRQ
ILRESGGIDKETMGFTYSGIRTNGATNACRRSGSSFYAEMKWLLSNTDNAAFPQMTK
SYKNTRRDPALIVWGVHHSGSTTEQTKLYGSGSKLITVGSSNYQQSFVPSPGARPQV
NGQSGRIDFHWLILDPNDTVTFSFNGAFIAPDRASFLRGKSMGIQSGVQVDANCEGD
CYHSGGTIISNLPFQNINSRAVGKCPRYVKQESLMLATGMKNVPELPKGRGLFGAIAG
FIENGWEGLIDGWYGFRHQNAQGEGTAADYKSTQSAIDQITGKLNRLIEKTNQQFELI
DNEFTEVEKQIGNVINWTRDSMTEVWSYNAELLVAMENQHTIDLTDSEMNKLYERV
RRQLRENAEEDGTGCFEIFHKCDDNCMASIRNNTYDHSKYREEAMQNRIQIDPVKLS
SGYKDVILWFSFGASCFILLAIAMGLVFICVKNGNMRCTICI*
SEQ ID NO. 17 (nucleotide sequence of the HA coding region of BP) 
ATGAAGGCCATCATCGTGCTGCTGATGGTGGTGACAAGCAACGCCGACAGAATCT
GCACCGGCATCACAAGCAGCAACAGCCCCCACGTGGTGAAGACCGCCACCCAAG
GCGAGGTGAACGTGACCGGCGTGATCCCCCTGACCACAACCCCCACCAAGAGCT
ACTTCGCCAACCTGAAGGGCACAAGAACAAGAGGCAAGCTGTGCCCCGACTGCC
TGAACTGCACCGACCTGGACGTGGCCCTGGGGAGACCCATGTGTGTGGGCACCA
CCCCTAGCGCCAAGGCTAGCATCCTGCACGAGGTGAGACCCGTGACAAGCGGCT
GCTTCCCCATCATGCACGACAGAACCAAGATCAGACAGCTGCCCAACCTGCTGAG
AGGCTACGAGAAGATCAGACTGTCCACACAGAACGTGATCGACGCTGAAAAGGC
TCCCGGCGGCCCCTACAGACTGGGCACAAGCGGCAGCTGCCCCAACGCCACAAG
CAAGATCGGCTTCTTCGCCACCATGGCCTGGGCCGTGCCCAAGGACAACTACAAG
AACGCCACCAACCCCCTGACCGTGGAGGTGCCCTACATCTGCACCGAGGGCGAG
GATCAGATCACCGTGTGGGGCTTCCACAGCGACGACAAGACACAGATGAAAAGC
CTGTACGGCGACAGCAACCCTCAGAAGTTCACAAGCAGCGCCAACGGCGTGACC
ACCCACTACGTGAGCCAAATCGGCGACTTCCCCGATCAGACCGAGGACGGCGGCC
TGCCTCAGAGCGGCAGAATCGTGGTGGACTACATGATGCAGAAGCCCGGCAAGA
CCGGCACCATCGTGTATCAGAGAGGCGTGCTGCTGCCTCAGAAGGTGTGGTGCGC
TAGCGGCAGAAGCAAGGTGATCAAGGGCAGCCTGCCCCTGATCGGCGAGGCCGA
CTGCCTGCACGAGGAGTACGGCGGCCTGAACAAGAGCAAGCCCTACTACACCGG
CAAGCACGCCAAGGCCATCGGCAACTGCCCCATCTGGGTGAAGACCCCCCTGAA
 
GCTGGCCAACGGCACCAAGTACAGACCCCCCGCCAAGCTGCTGAAGGAGAGAGG
CTTCTTCGGCGCCATCGCCGGCTTTCTCGAGGGGGGCTGGGAGGGCATGATCGCC
GGCTGGCATGGCTACACAAGCCACGGCGCCCACGGGGTCGCCGTCGCCGCCGAC
CTCAAGAGCACCCAAGAGGCCATCAACAAGATCACCAAGAACCTGAACAGCCTG
AGCGAGCTGGAGGTGAAGAACCTGCAGAGACTGAGCGGCGCCATGGACGAGCTG
CACAACGAGATCCTGGAGCTGGACGAGAAGGTGGACGACCTGAGAGCCGACACC
ATCAGCAGCCAAATCGAGCTGGCCGTGCTGCTGAGCAACGAGGGCATCATCAACA
GCGAGGACGAGCACCTGCTGGCCCTGGAGAGAAAGCTGAAGAAGATGCTGGGCC
CTAGCGCCGTGGACATCGGCAACGGCTGCTTCGAGACCAAGCACAAGTGCAATCA
GACCTGCCTGGACAGAATCGCCGCCGGCACCTTCAACGCCGGCGAGTTCAGCCTG
CCCACCTTCGACAGCCTGAACATCACCGCCGCTAGCCTGAACGACGACGGCCTGG
ACAACCACACCATCCTGCTGTACTACAGCACCGCTGCTAGCTCCCTCGCCGTGACC
CTGATGCTGGCCATCTTCATCGTGTACATGGTGAGCAGAGACAACGTGAGCTGCA
GCATCTGCCTGTGA
SEQ ID NO. 18 (amino acid sequence of the HA coding region of BP) 
MKAIIVLLMVVTSNADRICTGITSSNSPHVVKTATQGEVNVTGVIPLTTTPTKSYFANL
KGTRTRGKLCPDCLNCTDLDVALGRPMCVGTTPSAKASILHEVRPVTSGCFPIMHDR
TKIRQLPNLLRGYEKIRLSTQNVIDAEKAPGGPYRLGTSGSCPNATSKIGFFATMAWAV
PKDNYKNATNPLTVEVPYICTEGEDQITVWGFHSDDKTQMKSLYGDSNPQKFTSSAN
GVTTHYVSQIGDFPDQTEDGGLPQSGRIVVDYMMQKPGKTGTIVYQRGVLLPQKVW
CASGRSKVIKGSLPLIGEADCLHEEYGGLNKSKPYYTGKHAKAIGNCPIWVKTPLKL
ANGTKYRPPAKLLKERGFFGAIAGFLEGGWEGMIAGWHGYTSHGAHGVAVAADLKS
TQEAINKITKNLNSLSELEVKNLQRLSGAMDELHNEILELDEKVDDLRADTISSQIELA
VLLSNEGIINSEDEHLLALERKLKKMLGPSAVDIGNGCFETKHKCNQTCLDRIAAGTF
NAGEFSLPTFDSLNITAASLNDDGLDNHTILLYYSTAASSLAVTLMLAIFIVYMVSRDN
VSCSICL*
SEQ ID NO. 19 (nucleotide sequence of the M2 protein) 
ATGAGCCTGCTGACCGAAGTGGAAACCCCGACCCGCAGCGAATGGGAATGCCGC
TGCAGCGGCAGCAACGATCCGCTGGTGATTGCGGCGAACATTATTGGCATTCTGCA
TCTGATTCTGTGGATTACCGATCGCCTGTTTTTTAAATGCATTTATCGCCGCTTTAAA
TATGGCCTGAAACGCGGCCCGAGCACCGAAGGCGTGCCGGAAAGCATGCGCGAA
GAATATCAGCAAGAACAGCAGAGCGCGGTGGATGTGGATGATGGCCATTTTGTGA
ACATTGAACTGGAATAA
SEQ ID NO. 20 (amino acid sequence of the M2 protein) 
MSLLTEVETPTRSEWECRCSGSNDPLVIAANIIGILHLILWITDRLFFKCIYRRFKYGLK
RGPSTEGVPESMREEYQQEQQSAVDVDDGHFVNIELE*
SEQ ID NO. 21 (nucleotide sequence of the 51 chimeric antigen protein) 
ATGAAGGCCATCCTGGTGGTGATGCTGTACACCTTCACCACCGCCAACGCCGACA
CCCTGTGCATCGGCTACCACGCCAACAACAGCACCGACACCGTGGACACCGTGCT
GGAGAAGAACGTGACCGTGACCCACAGCGTGAACCTGCTGGAGGACAAGCACA
ACGGCAAGCTG
TGTGACCTGGACGGCGTGAAACCTCTGATCCTGAGAGACTGCAGCGTGGCCGGCT
GGCTGCTGGGCAACCCCATGTGCGACGAGTTCATCAACGTGCCCGAGTGGAGCTA
CATCGTGGAGAAGGCCAACCCCGTGAACGACCTGTGCTACCCCGGCGACTTCAAC
GACTACGAGGAGCTGAAGCACCTGCTGAGCAGAATCAACCACTTCGAGAAGATT
CAGATCATCCCCAAGAGCAGCTGGAGCAGCCACGAGGCTAGCCTGGGCGTGAGC
AGCGCCTGCCCCTACCAAGGCAAGAGCAGCTTCTTCAGAAACGTGGTGTGGCTG
ATCAACAAGAACAGCACCTACCCCACCATCAAGAGAAGCTACAACAACACCAAC
CAAGAGGACCTGCTGGTGCTGTGGGGCATCCACCACCCCAACGACGCCGCCGAG
CAGACCAAGCTGTATCAGAATCCTACCACCTACATCAGCGTGGGCACAAGCACCC
TGAATCAGAGACTGGTGCCTAGAATCGCCACAAGATCCAAGGTGAACGGGCAGA
GCGGCAGAATGGAGTTCTTCTGGACCATCCTGAAGCCTAACGACGCCATCAACTT
CGAGAGCAACGGCAACTTCATCGCCCCCGAGTACGCCTACAAGATCGTGAAGAA
GGGCGACAGCACCATCATGAAAAGCGAGCTGGAGTACGGCAACTGC
AACACCACCTGTCAGACCCCCGAGGGCGCCATCAACACAAGCCTGCCCTTTCAGA
ACGTGCACCCCATCACCATCGGCAAGTGCCCTAAGTACGTCAAGAGCACCAAGCT
GAGACTGGCCACCGGCCTGAGAAACGTGCCTAGCATTCAGAGCAGAGGCCTGTT
CGGCGCCATCGCCGGCTTCATCGAAGGCGGCTGGACCGGCATGGTGGACGGCTGG
TACGGCTACCACCATCAGAACGAGCAAGGCAGCGGCTACGCCGCCGATCTCAAGA
GCACACAGAACGCCATCGACAAGATCACCAACAAGGTGAACAGCGTGATCGAGA
AGATGAACACACAGTTCACCGCCGTGGGCAAGGAGTTCAACCACCTGGAGAAGA
GAATCGAGAACCTGAATAAAAAGGTGGACGACGGCTTCCTCGACATCTGGACCTA
CAACGCCGAGCTGCTGGTGCTGCTGGAGAACGAGAGAACCCTGGACTACCATGA
CAGCAACGTGAAGAACCTCTACGAGAAGGTGAGAAATCAGCTGAAGAACAACGC
CAAGGAGATCGGCAACGGGTGCTTCGAGTTCTACCACAAGTGCGACAACACCTG
CATGGAGAGCGTGAAGAACGGCACCTACGACTACCCCAAGTACAGCGAGGAGGC
CAAGCTGAACAGAGAGAAGATCGACGGCGTGAAGCTGGACAGCACAAGAATCTA
TCAGATCCTGGCCATCTACAGCACCGTGGCTAGCAGCCTGGTGCTCGTGGTGAGC
 
CTGGGCGCCATCAGCTTCTGGATGTGCAGCAATGGCAGCCTGCAGTGTCGGATCT
GCATC
SEQ ID NO. 22 (amino acid sequence of the 51 chimeric antigen protein) 
MKAILVVMLYTFTTANADTLCIGYHANNSTDTVDTVLEKNVTVTHSVNLLEDKHNG
KLCDLDGVKPLILRDCSVAGWLLGNPMCDEFINVPEWSYIVEKANPVNDLCYPGDFN
DYEELKHLLSRINHFEKIQIIPKSSWSSHEASLGVSSACPYQGKSSFFRNVVWLINKNS
TYPTIKRSYNNTNQEDLLVLWGIHHPNDAAEQTKLYQNPTTYISVGTSTLNQRLVPRI
ATRSKVNGQSGRMEFFWTILKPNDAINFESNGNFIAPEYAYKIVKKGDSTIMKSELEY
GNCNTTCQTPEGAINTSLPFQNVHPITIGKCPKYVKSTKLRLATGLRNVPSIQSRGLFG
AIAGFIEGGWTGMVDGWYGYHHQNEQGSGYAADLKSTQNAIDKITNKVNSVIEKM
NTQFTAVGKEFNHLEKRIENLNKKVDDGFLDIWTYNAELLVLLENERTLDYHDSNVK
NLYEKVRNQLKNNAKEIGNGCFEFYHKCDNTCMESVKNGTYDYPKYSEEAKLNRE
KIDGVKLDSTRIYQILAIYSTVASSLVLVVSLGAISFWMCSNGSLQCRICI
SEQ ID NO. 23 (5' UTR)
   ACTCTTCTGGTCCCCACAGACTCAGAGAGAACCCACC
SEQ ID NO. 24 (3' UTR) 
GCTGGAGCCTCGGTGGCCATGCTTCTTGCCCCTTGGGCCTCCCCCCAGCCCCTCCT
CCCCTTCCTGCACCCGTACCCCCGTGGTCTTTGAATAAAGTCTGAGTGGGCGGC
SEQ ID NO. 25 (Poly A)

AAAAAAAAAAAAAAAAAAAAAAAAAAAAAAAAAAAAAAAAAAAAAAAAAAAA
AAAAAAAAGATATCAAAAAAAAAAAAAAAAAAAGAAAAAAAAAAAAAAAAAA
AGAAAAAAAAAAAAAAAAA

## Claims

1. An mRNA, comprising a nucleotide sequence encoding a chimeric immunogenic polypeptide, wherein the chimeric immunogenic polypeptide comprises an immunogenic fragment of hemagglutinin HA of influenza A H5N1, an immunogenic fragment of hemagglutinin HA of influenza A H1N1, and an immunogenic fragment of hemagglutinin HA of influenza B Victoria linked together.

2. The mRNA of claim 1, wherein the immunogenic fragments comprised in the chimeric immunogenic polypeptide are linked together sequentially from the N-terminus to the C-terminus in an order selected from any one of the following:
(1) the immunogenic fragment of hemagglutinin HA of H5N1, the immunogenic fragment of hemagglutinin HA of H1N1, the immunogenic fragment of hemagglutinin HA of influenza B Victoria;
(2) the immunogenic fragment of hemagglutinin HA of H1N1, the immunogenic fragment of hemagglutinin HA of H5N1, the immunogenic fragment of hemagglutinin HA of influenza B Victoria;
(3) the immunogenic fragment of hemagglutinin HA of the influenza B Victoria, the immunogenic fragment of hemagglutinin HA of H5N1, the immunogenic fragment of hemagglutinin HA of H1N1; or
(4) the immunogenic fragment of hemagglutinin HA of influenza B Victoria, the immunogenic fragment of hemagglutinin HA of H1N1, the immunogenic fragment of hemagglutinin HA of H5N1.

3. The mRNA of claim 1 or 2, wherein the immunogenic fragments are linked directly or via a linker,
preferably, the immunogenic fragment of hemagglutinin HA of H5N1 and the immunogenic fragment of hemagglutinin HA of H1N1 are linked directly via a covalent bond; and
the immunogenic fragment of hemagglutinin HA of influenza B Victoria is linked to the immunogenic fragment of hemagglutinin HA of influenza A via a linker;
preferably, the linker is a peptide linker;
more preferably, the linker is a linker having a structure represented by -((G)ₙS)ₘ-, wherein n is selected from 1, 2, 3, 4, 5, or 6, and m is selected from 1, 2, 3, 4, 5, or 6; and
further preferably, the linker is a peptide linker having an amino acid sequence set forth in SEQ ID NO: 8.

4. The mRNA of any one of claims 1 to 3, wherein the immunogenic fragment of hemagglutinin HA of influenza A H5N1 is a head of HA, and the head of HA preferably comprises an amino acid sequence as set forth in SEQ ID NO: 4 or an amino acid sequence having at least 80%, 90%, 95%, 99% sequence identity to SEQ ID NO: 4;
optionally, the immunogenic fragment of hemagglutinin HA of influenza A H1N1 is a stem of HA, and the stem of HA preferably comprises an amino acid sequence as set forth in SEQ ID NO: 6 or an amino acid sequence having at least 80%, 90%, 95%, 99% sequence identity to SEQ ID NO: 6; and
optionally, the immunogenic fragment of hemagglutinin HA of influenza B Victoria comprises an amino acid sequence as set forth in SEQ ID NO: 10 or an amino acid sequence having at least 80%, 90%, 95%, 99% sequence identity to SEQ ID NO: 10.

5. The mRNA of any one of claims 1 to 4, wherein the chimeric immunogenic polypeptide comprises the head of hemagglutinin HA of influenza A H5N1, the stem of hemagglutinin HA of influenza A H1N1, and full-length hemagglutinin HA of influenza B Victoria linked together; and
preferably, the immunogenic polypeptide comprises an amino acid sequence as set forth in SEQ ID NO: 12 or an amino acid sequence having at least 80%, 90%, 95%, 99% sequence identity to SEQ ID NO: 12.

6. The mRNA of any one of claims 1 to 5, wherein the chimeric immunogenic polypeptide further comprises an N-terminal leader sequence, the leader sequence preferably comprises an amino acid sequence as set forth in SEQ ID NO: 2 or encoded by SEQ ID NO: 1, or an amino acid sequence having at least 80%, 90%, 95%, 99% sequence identity to a sequence set forth in SEQ ID NO: 2 or encoded by SEQ ID NO: 1, and is capable of promoting proper folding of the chimeric immunogenic polypeptide.

7. The mRNA of any one of claims 1 to 6, comprising a nucleotide sequence as set forth in SEQ ID NO: 11 or a nucleotide sequence having at least 80%, 90%, 95%, 99% sequence identity to SEQ ID NO: 11.

8. The mRNA of any one of claims 1 to 7, further comprising regulatory elements such as a 5' UTR, a 3' UTR, and a poly A tail operably linked to the encoding nucleotide sequence, and
preferably, the 5' UTR comprises a sequence as set forth in SEQ ID NO: 23 or a sequence having at least 80%, 90%, 95%, 99% sequence identity to SEQ ID NO: 23, the 3' UTR comprises a sequence as set forth in SEQ ID NO: 24 or a sequence having at least 80%, 90%, 95%, 99% sequence identity to SEQ ID NO: 24, and the poly A tail comprises a sequence as set forth in SEQ ID NO: 25 or a sequence having at least 80%, 90%, 95%, 99% sequence identity to SEQ ID NO: 25.

9. The mRNA of any one of claims 1 to 8, further comprising a 5' cap, wherein the 5' cap is preferably m7G(5')ppp(5')(2'-OMeA)pG.

10. The mRNA of any one of claims 1 to 9, further comprising a chemical modification, wherein the chemical modification is preferably a modification of all or part of uridylic acids in the nucleotide sequence to 1-methylpseudouridine.

11. A composition, comprising the mRNA of any one of claims 1 to 10.

12. The composition of claim 11, further comprising:
an mRNA encoding an immunogenic fragment of hemagglutinin HA of another influenza A virus subtype, preferably encoding an immunogenic fragment of hemagglutinin HA of H3N2 and/or H7N9 subtypes; and/or
an mRNA encoding an immunogenic fragment of hemagglutinin HA of another influenza B virus subtype, preferably encoding an immunogenic fragment of hemagglutinin HA of a B/Yamagata subtype.

13. The composition of claim 11 or 12, further comprising:
an mRNA encoding an immunogenic fragment of influenza virus matrix protein M1, influenza virus ion channel protein M2, and/or influenza virus nucleoprotein NP.

14. The composition of any one of claims 11 to 13, comprising:
a first mRNA selected from any one of claims 1 to 10;
a second mRNA encoding a second immunogenic polypeptide, wherein the second immunogenic polypeptide comprises the immunogenic fragment of hemagglutinin HA of H3N2;
a third mRNA encoding a third immunogenic polypeptide, wherein the third immunogenic polypeptide comprises the immunogenic fragment of hemagglutinin HA of H7N9; and
a fourth mRNA encoding a fourth immunogenic polypeptide, wherein the fourth immunogenic polypeptide comprises the immunogenic fragment of hemagglutinin HA of B/Yamagata.

15. The composition of claim 14, wherein a content ratio by mass of the first mRNA, the second mRNA, the third mRNA, and the fourth mRNA is in a range of (2-5):(2-5):(2-5):(0.5-5), preferably 5:5:2:0.5 or 5:5:2:2.5 or 5:2:2:0.5 or 5:5:5:5.

16. The composition of claim 14, further comprising:
a fifth mRNA encoding a fifth immunogenic polypeptide, wherein the fifth immunogenic polypeptide comprises the immunogenic fragment of influenza virus ion channel protein M2.

17. The composition of claim 16, wherein a content ratio by mass of the first mRNA, the second mRNA, the third mRNA, the fourth mRNA, and the fifth mRNA is in a range of (2-5):(2-5):(2-5):(0.5-5):(2-5), and is preferably selected from 5:5:5:5:5, 5:5:2:2:2, 5:2:2:2:2, 5:2:2:0.5:0.5, 5:5:2:0.5:2.5, or 5:2:2:0.5:3.

18. The composition of any one of claims 11 to 17, wherein the hemagglutinin antigen is recommended or selected according to standardized criteria used by the World Health Organization's Global Influenza Surveillance and Response System (GISRS).

19. The composition of any one of claims 11 to 18, wherein a sequence of the immunogenic fragment of hemagglutinin HA of H3N2 comprises an amino acid sequence set forth in SEQ ID NO: 14 or a sequence having at least 80%, 90%, 95%, 99% sequence identity to SEQ ID NO: 14, a sequence of the immunogenic fragment of hemagglutinin HA of H7N9 comprises an amino acid sequence set forth in SEQ ID NO: 16 or a sequence having at least 80%, 90%, 95%, 99% sequence identity to SEQ ID NO: 16, a sequence of the immunogenic fragment of hemagglutinin HA of B/Yamagata comprises an amino acid sequence set forth in SEQ ID NO: 18 or a sequence having at least 80%, 90%, 95%, 99% sequence identity to SEQ ID NO: 18; and/or a sequence of the immunogenic fragment of influenza virus ion channel protein M2 comprises an amino acid sequence set forth in SEQ ID NO: 20 or a sequence having at least 80%, 90%, 95%, 99% sequence identity to SEQ ID NO: 20.

20. The composition of any one of claims 11 to 19, wherein
the first mRNA comprises a nucleotide sequence as set forth in SEQ ID NO: 11 or a sequence having at least 80%, 90%, 95%, 99% sequence identity to SEQ ID NO: 11;
the second mRNA comprises a nucleotide sequence as set forth in SEQ ID NO: 13 or a sequence having at least 80%, 90%, 95%, 99% sequence identity to SEQ ID NO: 13;
the third mRNA comprises a nucleotide sequence as set forth in SEQ ID NO: 15 or a sequence having at least 80%, 90%, 95%, 99% sequence identity to SEQ ID NO: 15;
the fourth mRNA comprises a nucleotide sequence as set forth in SEQ ID NO: 17 or a sequence having at least 80%, 90%, 95%, 99% sequence identity to SEQ ID NO: 17; and/or
the fifth mRNA comprises a nucleotide sequence as set forth in SEQ ID NO: 19 or a sequence having at least 80%, 90%, 95%, 99% sequence identity to SEQ ID NO: 19.

21. The composition of any one of claims 11 to 20, wherein the mRNA further comprises regulatory elements such as a 5' UTR, a 3' UTR, and a poly A tail operably linked to an encoding polynucleotide sequence,
preferably, the 5' UTR comprises a sequence as set forth in SEQ ID NO: 23 or a sequence having at least 80%, 90%, 95%, 99% sequence identity to SEQ ID NO: 23, the 3' UTR comprises a sequence as set forth in SEQ ID NO: 24 or a sequence having at least 80%, 90%, 95%, 99% sequence identity to SEQ ID NO: 24, and the poly A tail comprises a sequence as set forth in SEQ ID NO: 25 or a sequence having at least 80%, 90%, 95%, 99% sequence identity to SEQ ID NO: 25.

22. The composition of any one of claims 11 to 21, further comprising a 5' cap, wherein the 5' cap is preferably m7G(5')ppp(5')(2'-OMeA)pG.

23. The composition of any one of claims 11 to 22, wherein the mRNA further comprises a chemical modification, and the chemical modification is preferably a modification of all or part of uridylic acids to 1-methylpseudouridine.

24. The composition of any one of claims 11 to 23, further comprising a pharmaceutically acceptable carrier, wherein preferably the carrier is a lipid nanoparticle, and the mRNA is encapsulated in one or more lipid nanoparticles.

25. The composition of any one of claims 11 to 24, wherein individual particles of the lipid nanoparticles encapsulate the first to fourth mRNAs in substantially the same ratio, or individual particles separately encapsulate the first to fourth mRNAs in different ratios, or individual particles separately encapsulate any one of the first, second, third, or fourth mRNA.

26. The composition of any one of claims 11 to 25, wherein individual particles of the lipid nanoparticles encapsulate the first to fifth mRNAs in substantially the same ratio, or individual particles separately encapsulate the first to fifth mRNAs in different ratios, or individual particles separately encapsulate any one of the first, second, third, fourth, or fifth mRNA.

27. The composition of any one of claims 11 to 26, wherein the lipid nanoparticles comprise an ionizable lipid, a phospholipid, a structural lipid, and a polyethylene glycol (PEG)-lipid, and
preferably, a molar ratio of the ionizable lipid, the phospholipid, the structural lipid, and the PEG-lipid is preferably (20-60):(5-25):(25-55):(0.5-5); and more preferably, the molar ratio of the ionizable lipid, the phospholipid, the structural lipid, and the PEG-lipid is (40-55):(5-15):(30-50):(1-3).

28. The composition of claim 27, wherein
the phospholipid is selected from one or two or more of the following compounds: dilauroyl phosphatidylcholine (DLPC), dimyristoyl phosphatidylcholine (DMPC), dioleoyl phosphatidylcholine (DOPC), dipalmitoyl phosphatidylcholine (DPPC), distearoyl phosphatidylcholine (DSPC), dioleoyl phosphatidylcholine (DUPC), palmitoyloleoyl phosphatidylcholine (POPC), 1,2-di-O-octadecyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-divinyl-sn-glycero-3-phosphocholine, 1,2-diarachidoyl-sn-glycero-3-phosphocholine, 1,2-dioleoyl-sn-glycero-3-phosphoethanolamine (DOPE), 1,2-distearoyl-sn-glycero-3-phosphoethanolamine, 1,2-bis(hydroxyvinyl)-sn-glycero-3-phosphoethanolamine, 1,2-divinyl-sn-glycero-3-phosphoethanolamine, 1,2-diarachidoyl-sn-glycero-3-phosphoethanolamine, 1,2-dithiohexaenoic acid-sn-glycero-3-phosphoethanolamine, 1,2-dioleoyl-sn-glycero-3-phospho-(1'-glycerol) sodium salt (DOPG), or sphingomyelin, and is preferably DSPC;
the structural lipid is selected from one or two or more of cholesterol, coprostanol, sitosterol, ergosterol, stigmasterol, and is preferably cholesterol; and/or
the PEG-lipid is selected from one or two or more of PEG-modified phosphatidylethanolamine, PEG-modified phosphatidic acid, PEG-modified ceramide, PEG-modified dialkylamine, PEG-modified diacylglycerol, or PEG-modified dialkylglycerol, and is preferably DMG-PEG2000.

29. The composition of any one of claims 11 to 28, wherein the composition is a vaccine.

30. A nucleic acid molecule, encoding the mRNA of any one of claims 1 to 10, wherein the nucleic acid molecule is preferably a DNA, and more preferably a DNA plasmid.

31. A fusion protein for preventing or treating influenza virus infection, comprising an amino acid sequence encoded by the mRNA of any one of claims 1 to 10.

32. A composition for preventing or treating influenza virus infection, comprising the fusion protein of claim 31.

33. A method for inducing an immune response against an influenza virus in a subject, comprising administering to the subject an effective dose of the mRNA of any one of claims 1 to 10, the composition of any one of claims 11 to 29 or 32, the nucleic acid molecule of claim 30, or the fusion protein of claim 31; and
preferably, the method comprises administering to the subject twice or three times.

34. Use of the mRNA of any one of claims 1 to 10, the composition of any one of claims 11 to 29 or 32, the nucleic acid molecule of claim 30, or the fusion protein of claim 31 in preparation of a medicament for preventing or treating influenza virus infection.

35. Use of the mRNA of any one of claims 1 to 10, the composition of any one of claims 11 to 29 or 32, the nucleic acid molecule of claim 30, or the fusion protein of claim 31 in preventing or treating influenza virus infection.
